# EUROPEAN PATENT SPECIFICATION

(11) **EP 2 843 037 B1**
(45) Date of publication and mention of the grant of the patent: **05.06.2019**
(21) Application number: 13782122.9
(22) Date of filing: 23.04.2013
(51) Int. Cl.: C12N 1/12, C12P 7/64, C12N 15/09, C12R 1/89

(54) **METHOD FOR CULTURING A MICROALGAE CAPABLE OF FORMING A BIOFILM ON THE SURFACE OF A LIQUID, AND RECOVERING BIOMASS AND OIL BOTH PRODUCED FROM SAID BIOFILM**
VERFAHREN ZUR KULTIVIERUNG VON MIKROALGEN, DIE AUF DER OBERFLÄCHE EINER FLÜSSIGKEIT EINER BIOFILM BILDEN, UND VERFAHREN ZUR ERFASSUNG HERGESTELLTE BIOMASSE UND ÖL AUS DIESEM BIOFILM
PROCÉDÉ DE CULTURE D'UNE MICRO-ALGUE CAPABLE DE FORMER UN BIOFILM SUR LA SURFACE D'UN LIQUIDE ET RÉCUPÉRATION DE LA BIOMASSE ET DE L'HUILE PRODUITES À PARTIR DUDIT BIOFILM

(30) Priority: 24.04.2012 JP 2012099188; 24.04.2012 JP 2012099189
(43) Date of publication of application: 04.03.2015
(73) Proprietor: FUJIFILM Corporation, Tokyo 106-8620 (JP)
(72) Inventor: KANEHARA, Hideyuki, Ashigarakami-gun Kanagawa 258-8577 (JP); MATSUNAGA, Tadashi, Fuchu-shi Tokyo 183-8538 (JP); TANAKA, Tsuyoshi, Fuchu-shi Tokyo 183-8538 (JP); TANAKA, Masayoshi, Meguro-ku Tokyo (JP)
(74) Representative: Hoffmann Eitle
(86) International application number: PCT/JP2013/061952
(87) International publication number: WO 2013/161832

(56) References cited:
- US-A1- 2009 087 889
- US-A1- 2009 087 889
- DATABASE EMBL [Online] 1 March 2004 (2004-03-01), "Botryococcus sp. UTEX 2629 18S rRNA gene (partial), ITS1, 5.8S rRNA gene, ITS2 and 28S rRNA gene (partial), strain UTEX 2629", XP002745720, retrieved from EBI accession no. EM_STD:AJ581914 Database accession no. AJ581914
- DATABASE EMBL [Online] 13 May 2003 (2003-05-13), "Characiopodium sp. Mary 9/21 T-3w 18S ribosomal RNA gene, partial sequence.", XP002745721, retrieved from EBI accession no. EM_STD:AY197622 Database accession no. AY197622
- DATABASE EMBL [Online] 12 February 1992 (1992-02-12), "Characium hindakii 18S ribosomal RNA gene.", XP002745722, retrieved from EBI accession no. EM_STD:M63000 Database accession no. M63000
- ZHANG Z. ET AL.: 'Biomarker evidence for the co-occurrence of three races (A, B and L) of Botryococcus braunii in E1 Junco Lake' ORGANIC GEOCHEMISTRY vol. 38, no. 9, September 2007, GALAPAGOS, pages 1459 - 1478, XP022209175
- EROGLU E. ET AL.: 'Hydrocarbon productivities in different Botryococcus strains: comparative methods in product quantification' J. APPL. PHYCOL. vol. 23, no. 4, August 2011, pages 763 - 775, XP019930209
- CHRISTENSON L. ET AL.: 'Production and harvesting of microalgae for wastewater treatment, biofuels, and bioproducts' BIOTECHNOL. ADV. vol. 29, no. 6, 2011, pages 686 - 702, XP028306405
- OZKAN A. ET AL.: 'Reduction of water and energy requirement of algae cultivation using an algae biofilm photobioreactor' BIORESOUR. TECHNOL. vol. 114, 28 March 2012, pages 542 - 548, XP028422616
- ELA EROGLU ET AL: "Hydrocarbon productivities in differentstrains: comparative methods in product quantification", JOURNAL OF APPLIED PHYCOLOGY, KLUWER ACADEMIC PUBLISHERS, DO, vol. 23, no. 4, 2 September 2010 (2010-09-02), pages 763-775, XP019930209, ISSN: 1573-5176, DOI: 10.1007/S10811-010-9577-8
- Hoda H. Senousy ET AL: "PHYLOGENETIC PLACEMENT OF BOTRYOCOCCUS BRAUNII (TREBOUXIOPHYCEAE) AND BOTRYOCOCCUS SUDETICUS ISOLATE UTEX 2629 (CHLOROPHYCEAE)1 : PHYLOGENY OF BOTRYOCOCCUS", JOURNAL OF PHYCOLOGY., vol. 40, no. 2, 12 March 2004 (2004-03-12) , pages 412-423, XP055474902, US ISSN: 0022-3646, DOI: 10.1046/j.1529-8817.2004.03173.x
- VAZQUEZ-DUHALT R: "LIGHT EFFECT ON NEUTRAL LIPID ACCUMULATION AND BIOMASS COMPOSITION OF BOTRYOCOCCUS-SUDETICUS CHLOROPHYCEAE", CRYPTOGAMIE. ALGOL, ELSEVIER, PARIS, FR, vol. 12, no. 2, 1 January 1991 (1991-01-01), pages 109-119, XP008138892, ISSN: 0181-1568

## Description

### Technical Field

The present disclosure relates to a method for culturing microalgae; a biofilm formed on a liquid surface by the culturing method; biomass and oil obtained from the biofilm, a method for collecting the biofilm; and a method for producing biomass fuel. According to the method for culturing the microalgae, it is possible to form a biofilm on a liquid surface, and therefore, the culturing method is useful in energy-related fields.

In addition, the present disclosure relates to microalgae capable of forming a biofilm on a liquid surface; the biofilm formed on the liquid surface using the microalgae; and biomass and oil obtained from the biofilm. The microalgae according to the present disclosure are microalgae forming a biofilm on a liquid surface, and therefore, these are useful in the energy-related fields. The invention is defined in the claims.

### Background Art

In recent years, there has been a problem that an increase in fuel prices due to use of a large amount of fossil fuels, or global warming due to the greenhouse effect caused by carbon dioxide released into the air due to the use of fossil fuels has progressed accompanying development of industrial activities. As means for solving these problems, there are growing expectations for utilization of microalgae having an ability of fixing carbon dioxide using light energy to convert it into hydrocarbon compounds, biodiesel (triglycerides), or the like. Various research, in which carbon dioxide is fixed using light energy by, for example, culturing microalgae to produce biomass such as biodiesel or hydrocarbon compounds, have already been conducted.

As an existence form of microorganisms such as microalgae, for example, there is mainly an existence form of producing viscous secretions to form a highly ordered structure, that is, a biofilm (microorganism aggregate or microorganism film) for protecting themselves from external attack, and such an existence form has recently been attracting great attention in the medical or environmental fields or the like. It is known that in a biofilm, when formed, microorganisms show behavior which is different from individual properties thereof, that is, show properties as an aggregate.

For example, the microorganisms form a physical barrier, which makes preying of a predatory organism on the microorganisms difficult compared to when the microorganisms independently exist and makes substitution of the microorganisms by other microorganisms difficult.

In addition, the biofilm is generally formed on a surface of a solid substance such as a surface of rock or a plant, and specific reports on formation of the biofilm on a liquid surface using microalgae have not been confirmed yet.

There are various problems in producing biomass using microalgae. Moreover, efficient methods of culturing and collecting microalgae, and extracting biomass such as oil have not been developed, and the cost of producing biomass is high. Therefore, the production thereof has not carried out on a commercial scale. One of the biggest reasons thereof is that there is no efficient method for collecting the microalgae. Specifically, microalgae are generally grown while floating in a liquid. Therefore, in order to use the microalgae as biomass, microalgae with an extremely dilute concentration need to be collected from a large amount of liquid. In addition, light energy is required for growth of the microalgae. Therefore, it is impossible to excessively increase the concentration of the microalgae existing in the liquid in order to secure sufficient irradiation with light. As a result, it is necessary to filter a large amount of water in order to collect the microalgae floating in the liquid. In addition, the microalgae are generally small in size, and thus are difficult to be filtered. Although a method for using a precipitant, a method for using a centrifugal separator, and a method for using microalgae as bait for larger organisms and collecting the larger organisms have been attempted in order to study collecting methods for solving such problems, any methods have not led to a basic solution.

Due to the above-described reasons, it is desirable that microalgae be grown on a liquid surface in order to efficiently and simply collect the microalgae at a low cost.

Examples of floating of microalgae on a liquid surface include blooming (massive proliferation) of Microcystis and Botryococcus that occurs naturally, and red tide that occurs in the ocean. However, all of these are phenomena that occur in nature, and various kinds and a large number of impurities are mixed with microalgae generated on a liquid surface. Therefore, it is not certain whether pure microalgae grow on the liquid surface. Microcystis is referred to as an aggregate which is formed of microalgae that are not subjected to microorganism purification, that is, formed of various microalgae; which floats on a liquid surface; and which is formed of mainly powder-like microalgae as is referred to as blue-green algae. Therefore, Microcystis is different from the biofilm according to the present invention.

In regards to culturing of Botryococcus, Non-Patent Documents 1 and 2 show that Botryococcus exhibits floating properties on a liquid surface when oil accumulates. In addition, Patent Document 1 discloses in the Abstract that Botryococcus floats on a liquid surface. However, in Patent Document 1, Botryococcus is cultured on a surface of a hygroscopic cloth based on a phenomenon that the accumulation rate of hydrocarbon compounds is improved through direct contact between carbon dioxide and algae, and there is no disclosure in Examples in Patent Document 1 that Botryococcus directly floats on a liquid surface.

Non-Patent Document 3 discloses Botryococcus sp. UTEX2629 strains as Botryococcus sudeticus. However, the strains have a greater specific gravity than those of Botryococcus braunii and there is no disclosure relating to floating on a liquid surface.

As described above, there is no disclosure of specific modes in which Botryococcus actually floats on a liquid surface in the related art while there is a suggestion that Botryococcus floats on a liquid surface. Needless to say, there is no disclosure of formation of a biofilm on a liquid surface.

In addition, Non-Patent Document 4 discloses that Crypthecodinium cohnii (C. cohnii), which is cultured, floats on a liquid surface. However, there is no disclosure of formation of a biofilm on a liquid surface.

Non-Patent Document 6 relates to hydrocarbon productivities in different *Botryococcus* strains, in particular to comparative methods in product quantification. Non-Patent Document 7 relates to growth and production of cell constituents in batch cultures of *Botryococcus sudeticus*: the growth of, and the production of neutral lipids, carbohydrates and proteins by the alga *Botryococcus sudeticus* in batch cultures is described. Some physiological characteristics of the species are also discussed.

### Related Art

### Patent Document

[Patent Document 1] US-A-2009-0087889

### Non-Patent Document

[Non-Patent Document 1] The Ecology of Cyanobacteria, Their Diversity in Time and Space, B. A. Whitton & M. Potts, Eds, Kluwer Academic (1999), pp. 160
[Non-Patent Document 2] Oceanological Studies, 1998, Vol. 27, No. 1, Publisher: Index Copernicus p. 17
[Non-Patent Document 3] Melis et al., J apply Phycol (2010)
[Non-Patent Document 4] "Growth Inhibition of Dinoflagellate Algae in Shake Flasks: Not Due to Shear This Time", Biotechnol. Prog., 2010, Vol. 26, No. 1, pages 79-87
[Non-Patent Document 5] Journal of the Agricultural Engineering Society, Japan, pp. 971-975, Vol. 56 (10), 1988
[Non-Patent Document 6] Eroglu, E., et al., J. Appl. Phycol: Vol. 23 No. 4, August 2011, pages 763-775.
[Non-Patent Document 7] Vayquez-Duhalt, R. et al., Phytochemistry, Vol. 26, No. 4, 1 January 1987, pages 885-889

### Summary of the Invention

### Problems that the Invention is to Solve

Culturing microorganisms such as microalgae and extracting biomass such as biodiesel or hydrocarbon compounds from the cultured microorganisms have been attracting great attention. However, production thereof has not yet been carried out on a commercial scale. One of the reasons for this is that the production cost thereof is too high compared to energy sources derived from fossils which can be obtained from oil fields or the like. Primary causes of the increased production cost are that the cost for collecting microalgae which are dispersed in a liquid and are small in size is high; a large area is generally required for producing biomass and there is no method for uniformly introducing carbon dioxide to such a large area at a low cost; the installation cost is high because of extremely long-distance piping; the production output of biomass of algae per unit of light receiving surface is small; and the apparatus cost and operation cost for stirring a liquid medium in which algae are dispersed is high.

In addition, in the culturing of the microalgae in the related art, it is necessary to handle a large amount of liquid medium. For this reason, a large amount of energy is required. Furthermore, a large amount of water is necessary in order to supply a large amount of liquid medium.

Furthermore, according to NPL 5, it has been reported that the collection rate of Microcystis which has proliferated in water and on the surface of water is 80% and the water content ratio of concentrated Microcystis which was separated is 97%. A higher collection rate and a lower water content ratio of the collected substance are required in order to reduce the production cost.

There are various problems as described above in culturing microorganisms such as microalgae on a commercial scale and in producing a biomass product at a low cost. Among these, in the present invention, reducing the collection cost by facilitating the collection of small microalgae; reducing the supply cost of carbon dioxide (that is, reduction of installation cost of piping for carbon dioxide); eliminating the necessity of using a large amount of water with respect to the area of a liquid surface and eliminating the necessity of handling a large amount of liquid medium; increasing the collection rate of microalgae; and reducing the water content ratio of the collected microalgae exist as problems to be solved.

That is, an object of the present invention is to provide a method for culturing microalgae in which microalgae capable of forming a biofilm on a liquid surface are cultured such that the biofilm is formed on a liquid surface of a liquid medium.

In addition, another object of the present invention is to provide a biofilm formed on a liquid surface by the culturing method; biomass and oil obtained from the biofilm; a method for collecting the biofilm; and a method for producing biomass fuel.

In addition, still another object of the present invention is to provide microalgae having properties for solving the problems in the above-described related art, and specifically, microalgae capable of forming a biofilm on a liquid surface.

In addition, still another object of the present invention is to provide a biofilm formed on a liquid surface using microalgae according to the present invention; and biomass and oil obtained from the biofilm.

### Means for Solving the Problems

The present inventors have conducted extensive studies in order to solve the above-described problems, and as a result, they have found that certain kinds of microalgae proliferate while forming a biofilm on a liquid surface by culturing the microalgae under specific conditions. In addition, they have found that the biofilm formed in this manner can be easily collected using a slide glass substrate, a plastic film substrate, or the like. In addition, they have found that the biofilm formed on a liquid surface can be collected at a high collection rate and its water content ratio is low. Furthermore, they have found that oil can be obtained from the collected biofilm in this manner and the biofilm according to the present invention is useful as biomass.

In addition, the present inventors have conducted extensive studies in order to solve the above-described problems, and as a result, they have found that certain kinds of microalgae proliferate while forming a biofilm on a liquid surface. In addition, they have found that the biofilm formed in this manner can be easily collected using a slide glass substrate, a plastic film substrate, or the like. Furthermore, they have found that oil can be obtained from the collected biofilm in this manner and the biofilm according to the present invention is useful as biomass. The present invention has been completed based on such knowledge and provides, *inter alia,* a method for culturing microalgae, comprising:
culturing microalgae capable of forming a biofilm on a liquid surface such that the biofilm is formed on a liquid surface of a liquid medium, wherein the homology of the microalgae with base sequences of a region having greater than or equal to 1000 bases corresponding to *Botryococcus sudeticus* 18S rRNA sequence SEQ ID NO:1 is 95.0% to 99.9%. or the microalgae are Botryococcus sudeticus AVFF007 strain (deposition number FERM BP-11420).

Further aspects of the invention are defined in the claims.
[1] The method for culturing microalgae as described in any one of claims 1-5. A culture temperature can be higher than or equal to 0°C and less than 40°C.
   The water depth of the liquid medium can be greater than or equal to 0.4 cm.
   The water depth of the liquid medium can be 2.0 cm to 1 m.
   The water content ratio of the biofilm after collection is less than or equal to 95 mass%.
[2] A biofilm formed on a liquid surface by the method for culturing the microalgae as described in claim 6.
[3] Microalgae capable of forming a biofilm on a liquid surface. as defined in claim 7.
   wherein the homology of the microalgae with base sequences of a partial region corresponding to Botryococcus sudeticus among base sequences encoding a gene region of 18S rRNA is 95.0% to 99.9%.
   wherein the microalgae are Botryococcus sudeticus AVFF007 strains (deposition number of FERM BP-11420).
   The microalgae as described in claim 7, wherein the proliferation rate in a logarithmic growth phase of the microalgae on a liquid surface is higher than or equal to 5 g/m²/day by dry weight.
   wherein the water content ratio of the biofilm after collection is less than or equal to 95 mass%.
[4] A metod for collecting a biofilm as described in claim 9,
   wherein the biofilm formed on the liquid surface according to claim 6 is collected by depositing the biofilm on a substrate.
[5] A method for collecting a biofilm as described in claim 10,
   wherein the biofilm formed on the liquid surface according to claim 6 is collected by transferring the biofilm onto a substrate.
[6] A method for producing biomass fuel as described in claim 11, wherein the biofilm collected by the method for collecting the biofilm according to any one of claims 9 and 10 is used as fuel.

### Advantage of the Invention

According to the method for culturing microalgae according to the present invention, it is possible to form a biofilm on a liquid surface, and it is possible to extremely easily collect microalgae and to collect microalgae at a high collection rate compared to the floating culture in the related art. That is, in the present invention, in the stage of collecting the microalgae, the biofilm which is formed of aggregates of microalgae floats on the liquid surface, and the biofilm is set as a target to be collected. Therefore, it is unnecessary to collect microalgae from a large amount of medium as in the related art. Moreover, only the biofilm on the liquid surface and water contained in the biofilm are set as a target to be collected. Therefore, it is possible to greatly reduce the cost for collection. In addition, it is possible to reduce the water content ratio of the collected biofilm. Furthermore, it is unnecessary to handle a large amount of liquid medium and to use a large amount of water.

According to the microalgae capable of forming a biofilm on a liquid surface according to the present invention, it is possible to perform culturing on the liquid surface, and it is extremely easy to collect the microalgae compared to the flotation culture method in the related art. That is, in the present invention, in the stage of collecting the microalgae, the biofilm which is formed of aggregates of microalgae floats on the liquid surface, and the biofilm is set as a target to be collected. Therefore, it is unnecessary to collect microalgae from a large amount of medium as in the related art. Moreover, only the biofilm on the liquid surface and water contained in the biofilm are set as a target to be collected. Therefore, it is possible to greatly improve the collection efficiency. Accordingly, it is considered that it is possible to greatly reduce the production cost of biomass. In addition, the water content ratio of the collected biofilm is made to be the same as or lower than that in the method of the related art. Therefore, the efficiency of a biomass extraction process such as a drying process of an algal body in an oil extraction process is improved.

### Brief Description of Drawings

Fig. 1 is a composition of a C medium.
Fig. 2 is a composition of a CSi medium.
Fig. 3 is a view showing a microphotograph (magnification of 40) of AVFF007 strains.
Fig. 4 is a base sequence (SEQ ID No: 1) of the AVFF007 strains used in BLAST analysis.
Fig. 5 a systemic diagram of the Botryococcus sudeticus AVFF007 strains of microalgae.
Fig. 6 is a composition of an IMK medium.
Fig. 7 is a view showing a microphotograph (magnification of 40) of ASFF001 strains.
Fig. 8 is a base sequence (SEQ ID No: 2) of the ASFF001 strains used in BLAST analysis.
Fig. 9 is a view showing a microphotograph (magnification of 40) of AVFF004 strains.
Fig. 10 is a base sequence (SEQ ID No: 3) of the AVFF004 strains used in BLAST analysis.
Fig. 11 is a schematic view showing an example of a method for culturing microalgae of the present invention.
Fig. 12 is a state where a biofilm which is formed on a liquid surface and is configured of microalgae (film-like structure in Fig. 12) is collected using a second substrate (glass substrate).
Fig. 13 is a state of a biofilm deposited on the second substrate (glass substrate) after collecting the biofilm formed on the liquid surface of Fig. 12.
Fig. 14 is a view showing a photograph of liquid surface-floating culture.
Fig. 15 is a view of a microphotograph in a process in which the AVFF007 strains form the film-like structure on a liquid surface (left side: magnification of 4, right side: magnification of 40). The numerical value disclosed in the right side shows the number of days of culturing.
Fig. 16 is a composition of a CSiFF04 medium.
Fig. 17 a view of showing a photograph of a biofilm (three-dimensional structure) formed on a liquid surface in Example 2.
Fig. 18 is a view showing a relationship between the number of days of liquid surface-floating culture in Example 2 and the quantity of dry alga bodies of the biofilm for each number of days of liquid surface-floating culture.
Fig. 19 is a view showing a relationship between the number of days of liquid surface-floating culture in Example 2 and the maximum height (cm) of the biofilm for each number of days of liquid surface-floating culture.
Fig. 20 is a view showing a relationship between the number of days of liquid surface-floating culture in Example 2 and the water content ratio of the biofilm for each number of days of liquid surface-floating culture.
Fig. 21 is an analysis result of an oil component obtained from a biofilm formed in Example 4.
Fig. 22 is a view showing a state ((a) of Fig. 21) of a biofilm formed on a liquid surface in Example 5 and a state ((b) of Fig. 21) of a liquid surface after collecting the biofilm.
Fig. 23 is a composition of a CSiFF01 medium.
Fig. 24 is a view showing a relationship between the temperature for liquid surface-floating culture in Example 6 and the quantity ratio of dry alga bodies (quantity of dry alga bodies of the biofilm when the variable temperature culture at 23°C was regarded as a standard) in a biofilm at each temperature for liquid surface-floating culture.
Fig. 25 is a view showing a relationship between the water depth of media and the quantity of dry alga bodies of biofilms at each water depth of the media.
Fig. 26 is a view showing a relationship between the number of days of culturing in each concentration of a medium and the amount of light, and the quantity of dry alga bodies of a biofilm.
Fig. 27 is a view showing a relationship between the amount of light in each concentration of a medium and the quantity of dry alga bodies of a biofilm.
Fig. 28 is a view showing a relationship between various nitrate sources and the quantity of dry alga bodies of a biofilm for the numbers of days of culturing of 7 days and 14 days.
Fig. 29 is a view showing a relationship between the concentration of calcium and the quantity of dry alga bodies of a biofilm formed on a liquid surface for the numbers of days of culturing of 7 days and 14 days.
Fig. 30 is a composition of a CSiFF03 medium.
Fig. 31 is a view showing a relationship between the pH and the quantity of dry alga bodies of a biofilm formed on a liquid surface.
Fig. 32 is a view showing a relationship between the CO₂ concentration in a gas phase and the number of algal bodies of a biofilm formed on a liquid surface.
Fig. 33 is a result of observing culturing of microalgae on a liquid surface when various microalgal species are used.
Fig. 34 is a result of microscopically observing (magnification of 4) culturing on a liquid surface in the case of the AVFF004 strains, and is an example of liquid surface-floating culture ++.
Fig. 35 is a result of microscopically observing (magnification of 4) culturing on a liquid surface in a case of NIES2249 strains, and is an example of liquid surface-floating culture +++.
Fig. 36 is a result of microscopically observing (magnification of 4) culturing on a liquid surface in the case of the AVFF007 strains, and is an example of liquid surface-floating culture ++++.
Fig. 37 is a view showing a relationship between the amount of light and the quantity of dry alga bodies of a biofilm formed on a liquid surface.
Fig. 38 is a view showing an influence of shaking on liquid surface-floating culture.

### Mode for Carrying Out the Invention

Hereinafter, methods for culturing microalgae will be described in detail.

The method for culturing microalgae described herein is culturing microalgae capable of forming a biofilm on a liquid surface such that the biofilm is formed on a liquid surface of a liquid medium.

Unlike the related art of collecting microalgae which are dispersed in a liquid medium and are extremely small in size, according to the present invention, a biofilm formed on a liquid surface can be set as a target to be collected. Therefore, the size thereof for collection is extremely large compared to the former case and the collecting thereof is easy. Furthermore, a large amount of water remains even after collecting the microalgae in the case of the floating culture in the liquid medium in the related art. Moreover, it is necessary to perform high-cost operations such as a centrifugal operation and an evaporation operation in order to remove the water from the microalgae. However, in the method of the present invention, the biofilm, which has a lower water content ratio compared to the method of the related art because it is formed on a liquid surface, can be set as a target to be collected. Therefore, the necessity of such operations can be eliminated or the operations can be made simple compared to the method of the related art.

In addition, in the method of the present invention, the culturing is performed on a gas-liquid interface, and it thus shows low death of algal bodies due to drying.

### [Microalgae Capable of Forming Biofilm on Liquid Surface]

Microalgae capable of forming a biofilm on a liquid surface in the present invention have an ability of forming a biofilm on a liquid surface.

The microalgae referred to herein indicate minute algae of which individual existences cannot be identified with the human naked eye. The classification of microalgae is not particularly limited as long as the microalgae conform to the definition in the claims and thus have the ability of forming a biofilm on a liquid surface.

The microalgae are limited as defined in the claims, and any such microalgae can be appropriately used depending on the purpose. Examples of microalgae disclosed herein include the division Cyanophyta, the division Claucophyta, the division Rhodophyta, the division Chlorophyta, the division Cryptophyta, the division Haptophyta, the division Heterokontophyta, the division Dinophyta, the division Euglenophyta, and the division Chlorarachniophyta.

Among these, as the microalgae, diatoms of the division Heterokontophyta and the division Chlorophyta are advantageous, and the genus Botryococcus as defined in the claims is advantageous in terms of production of biomass.

The method for obtaining microalgae is not particularly limited, and any method can be appropriately selected depending on the purpose. Examples thereof include a method for collecting microalgae in nature, a method for using a commercially available product, and a method for obtaining microalgae from a culture collection or a depositary institution.

The biofilm referred to herein is a film-like structure or a steric three-dimensional structure, to be described later, which is formed of microorganisms, and generally indicates a microorganism structure (microorganism aggregate or microorganism film) which is adhered to the surface of rock or plastic. Besides these, the biofilm is also regarded as a film-like structure or a steric three-dimensional structure, to be described later, which is formed of microorganisms existing on the surface such as a liquid surface having fluidity in the present invention. In general, a biofilm particularly in nature also contains debris or pieces of plants besides microorganisms, and the biofilm of the present invention may also contain this. A subject is not intentionally set to a sample containing this in the present invention, but it is impossible to avoid the mixing-in of matter other than the target microorganisms in environments such as the outdoors which are open. However, it is preferable that the biofilm do not contain impurities such as debris or pieces of plants in view of the efficiency of collecting microalgae. Ideally, it is more preferable that the biofilm be formed of only the microalgae according to the present invention, a substance such as a matrix between cells which is secreted during the proliferation of the microalgae, and the like. In addition, in the present invention, it is preferable that the biofilm be configured such that individual microalgae are adhered to each other directly or via a substance such as the matrix between cells.

It is preferable that the microalgae capable of forming a biofilm on a liquid surface in the present invention be Botryococcus sudeticus microalgae. The
homology of the microalgae according to the present invention with base sequences of a partial region corresponding to Botryococcus sudeticus among base sequences encoding a gene region of 18S rRNA is 95.0% to 99.9%. The "partial region" referred to herein means a region having greater than or equal to 1000 base sequences. When testing the homology, use of every base sequence results in high reliability for the test of the homology. However, determining every base sequence is technically and financially difficult except for an extremely small number of species of organisms. In addition, only a specific portion (specifically, the vicinity of base sequences corresponding to base sequences of the AVFF007 strains which are set as a comparison target to be described later) of the base sequences of Botryococcus sudeticus has been reported. Furthermore, in general, it is considered that there can be attribution if about 1000 base sequences are read. From the above, the homology has been tested by the comparison with the base sequences of a "partial region" in the present invention, and it is considered that the reliability is sufficiently high.

In addition, the microalgae according to the present invention preferably have a high proliferation rate on a liquid surface. Specifically, the proliferation rate in a logarithmic growth phase of microalgae on a liquid surface (that is, an average proliferation rate per day during the period of the logarithmic growth phase) is preferably higher than or equal to 0.1 g/m²/day by dry weight, more preferably higher than or equal to 1 g/m²/day by dry weight, still more preferably higher than or equal to 5 g/m²/day by dry weight, and particularly preferably higher than or equal to 10 g/m²/day by dry weight. The proliferation rate in the logarithmic growth phase of the microalgae on the liquid surface is generally lower than or equal to 1000 g/m²/day by dry weight.

Particularly, the microalgae according to the present invention are more preferably Botryococcus sudeticus AVFF007 strains of microalgae (hereinafter, simply referred to as AVFF007 strains) from the viewpoints of good culturing on a liquid surface and a property of good recovery from the liquid surface; possession of a high proliferation rate; a high content of oil; little odor at least during culturing; and no generation of poisonous substances being confirmed.

The AVFF007 strains as microalgae used in Examples of the present specification are internationally deposited at the Patent Organism Depositary of the National Institute of Technology and Evaluation (Room No. 120, 2-5-8 Kazusakamatari Kisarazu-shi, Chiba-ken, Japan) dated September 28, 2011 with a deposition number of FERM BP-11420 [or at Patent Microorganism Depository of the National Institute of Advanced Industrial Science and Technology during international deposition (Central 6, 1-1, Higashi 1-chome, Tsukuba-shi, Ibaraki-ken, Japan)] under the Budapest Treaty.

The AVFF007 strains are new strains of fresh water microalgae which were isolated from a pond in Kyoto by the present inventors and belong to the genus Botryococcus and the species sudeticus.

Hereinafter, the method for isolating the microalgae (hereinafter, referred to as microorganism purification) and circumstances in which it has been determined that the AVFF007 strains of the microalgae are new strains will be described.

### (Microorganism Purification of AVFF007 Strains of Microalgae)

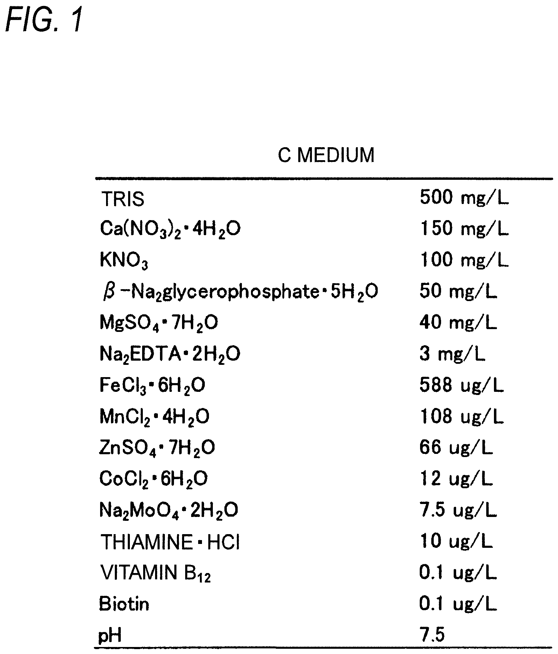

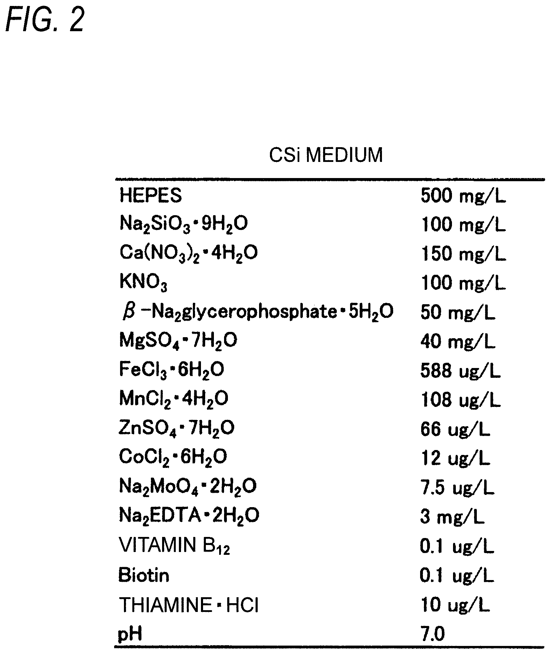

5 mL of natural fresh water was collected from a pond in Kyoto by putting it into a tube for homogenizing (TM-655S, Tomy Seiko Co., Ltd.). 100 µL of the collected natural fresh water was added to a 24 hole plate (microorganism culture plate 1-8355-02, As One Corporation) into which 1.9 mL of a medium, in which a C medium shown in Fig. 1 and a CSi medium shown in Fig. 2 were mixed at 1:1 (volume ratio), was put. The plate was provided in a plant bioshelf for tissue culture (AV152261-12-2, Ikeda Scientific Co., Ltd.) and was cultured at 23°C under continuous irradiation with 4000 lux light. After approximately one month, a yellow aggregate was generated in wells of the 24 hole plate. The aggregate was observed using an optical microscope and it was confirmed that there are a large number of microorganisms.

1 g of agarose (Invitrogen, UltraPure™ Agarose) was weighed out, and 200 mL of a medium in which a C medium and a CSi medium were mixed at 1:1 (volume ratio) was put into a 500 mL conical flask. The medium was subjected to an autoclave treatment for 10 minutes at 121°C, and approximately 20 mL of the medium at a time was added to an Azunol Petri dish (1-8549-04, As One Corporation) in a clean bench before being cooled and hardened to produce agarose gel.

The solution containing the microalgae in the 24 hole plate was diluted, and the solution was made to adhere to a loop portion of a disposable stick (1-4633-12, As One Corporation) and was applied on the prepared agarose gel to prepare a Petri dish in which the microalgae were applied on the agarose gel.

The Petri dish was placed on a plant bioshelf for tissue culture and was cultured at 23°C under continuous irradiation with 4000 lux light. After approximately 2 weeks, a green colony appeared on the agarose gel. The colony was adhered to a tip end of a sterilized bamboo skewer (1-5980-01, As One Corporation), and then, was suspended in the wells of the 24 hole plate into each of which 2 mL of the medium, in which the C medium and the CSi medium were mixed at 1:1 (volume ratio), was put. The 24 hole plate containing microalgae prepared in this manner was provided in a plant bioshelf for tissue culture and was cultured at 23°C under continuous irradiation with 4000 lux light. After approximately 2 weeks, the aqueous solution in the wells exhibited a green color. A small amount of solution was collected from all of the wells, the microalgae were observed using an optical microscope, and it was concluded that microorganism purification had been performed in wells, in which it was considered that there was only a single microalga.

The compositions of the C medium and the CSi medium were as shown in Figs. 1 and 2. For both the media, 900 mL of distilled water was subjected to an autoclave treatment for 10 minutes at 121°C and 100 mL of a C medium or a CSi medium at 10 times concentration was prepared. Then, the prepared medium was mixed with a solution which was sterilized using a filter having a pore size of 0.45 µm.

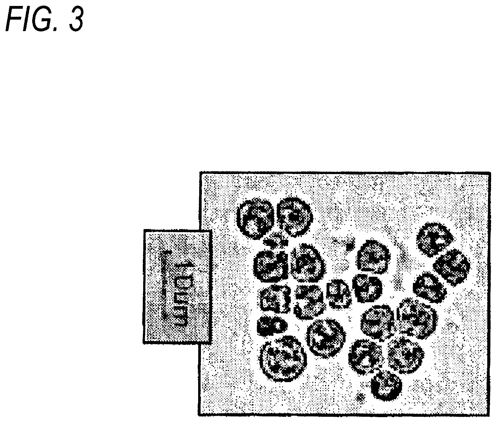

In addition, a view showing a microphotograph of AVFF007 strains at magnification of 40 is shown in Fig. 3.

### (Morphological Properties)

- All microalgae sink to the bottom surface if the microalgae are left for a while after performing a dispersion treatment.
- If the microalgae are cultured for a while, microalgae floating on a liquid surface appear. Accordingly, microalgae are divided into microalgae having sunk to the bottom surface and microalgae floating on the liquid surface. If further is performed continuously, a film-like structure appears on the liquid surface. If the culturing is performed further, a three-dimensional structure appears.
- Microalgae floating on the liquid surface respectively have a greater diameter and the average particle size thereof is 22.1 µm. Microalgae having sunk to the bottom surface have a smaller diameter compared to that of the microalgae floating on the liquid surface and the average particle size thereof is 7.8 µm.
- The microalgae on the liquid surface and on the bottom surface have a spherical shape and have different size distributions.
- The microalgae have cohesiveness and form a large colony.
- The microalgae are green and the color thereof turns to yellow in accordance with the progress of the culturing.
- There is little odor in collected substances and during the culturing, but in some cases, there is a smell like fresh vegetables. There is a smell like sulfur when a solvent is removed from the recovered substances.

### (Culturing Properties)

- The microalgae grow in fresh water whereas the proliferation in seawater becomes extremely slow. Only several % of seawater being mixed therein has an influence on the proliferation rate.
- During the cell proliferation, the cells proliferate through zoospores. Several zoospores to several tens of zoospores are generated from one zoospore.
- It is possible to perform photoautotrophic culture through photosynthesis.
- Nitrogen, phosphorus, potassium, calcium, magnesium, sulfur, manganese, and iron are essential for proliferation. In addition, inclusion of zinc, cobalt, molybdenum, and boron makes the proliferation favorable. Addition of vitamins also promotes the proliferation.

### (Physiological Properties)

- The growth temperature is less than or equal to 37°C. The higher the temperature, the better the proliferating properties are.
- The microalgae mostly do not proliferate at 40°C, but can withstand at least several hours under an environment of 40°C.
- The growth pH is 5 to 9. In some cases, the pH after the growth becomes higher than or equal to 8, for example, 10.5 depending on the kinds of media.
- If light or heat is provided, it is easy to generate carotenoid.
- Oil accumulates in a bacterial cell at 15 wt% to 30 wt% in terms of dry weight propertion.
- Algal bodies floating on the liquid surface have higher oil content than that of algal bodies having sunk to the bottom surface.
- Main components of the oil are hydrocarbon compounds and fatty acids. The fatty acids mainly include C16:0, C16:1, C18:1, and C18:2. The hydrocarbon compounds mainly include C17 and C21.
- AVFF007 strains dyed with Nile Red were observed by a fluorescence microscope. Then, it was confirmed that there was oil which was colored with the Nile Red as a bright fluorescent light-emitting region in microalgae in a fluorescent visual field. The oil can accumulates in a wide region within an alga body cell.
- A culture solution containing the AVFF007 strains was added dropwise to a slide glass, covered with a cover glass, and observed with a microscope. Then, oil-like oil droplets were released from the AVFF007 strains.
- Algal bodies floating on the liquid surface have a lower specific gravity than that of algal bodies having sunk to the bottom surface, but have a higher specific gravity than that of water.
- The amount of light at which it is possible to suitably perform proliferation is 200 µmol/m²/s to 800 µmol/m²/s. However, even at about 40 µmol/m²/s or at about 1500 µmol/m²/s, it is possible to perform the proliferation at a half proliferation rate compared to the suitable amount of light.

Identification of the AVFF007 strains was further performed in accordance with the following method (identification of the AVFF007 strains of microalgae).

The culturing method of the AVFF007 strains was as follows. 50 mL of a CSi medium was introduced to a conical flask with a 100 mL capacity, 0.5 mL of an AVFF007 strain solution at a concentration of 1000 x 10⁴ cells/mL was added thereto, and shaking culture was performed under light irradiation for 14 days at 25°C.

In order to obtain a dry powder of the AVFF007 strains, centrifugal operation was performed on 40 mL of the medium containing the AVFF007 strains obtained as described above using a centrifuge (MX-300 (Tomy Seiko Co., Ltd.) for 10 minutes at a centrifugal force of 6000 x g below 4°C. After removing a supernatant, the solid body was frozen together with the container using liquid nitrogen. Then, the total quantity of frozen solid body was transferred to a mortar which was chilled in advance using liquid nitrogen, and was ground using a pestle which was chilled in advance using liquid nitrogen.

The extraction of DNA from the microalgae was performed using DNeasy Plant Mini Kit (manufactured by Qiagen) according to the described manual. The purity and the amount of the extracted DNA were measured using e-spect (manufactured by Malcom Co., Ltd.). It was confirmed that the extracted DNA achieved the index of a purification degree which is A₂₆₀ₙₘ/A₂₈₀ₙₘ = 1.8 or greater, and about 5 ng/µL of DNA was taken.

There was no problem in the purification degree of the extracted DNA, and thus, a sample for PCR was prepared by diluting the DNA 10⁴ times in ultrapure water. An 18S rRNA gene region (rDNA region) was used as the sample for PCR. A cycle including 10 seconds at 98°C, 50 seconds at 60°C, and 10 seconds at 72°C was performed 30 times for the PCR using a GeneAmp PCR System 9700 (manufactured by Applied Biosystems). An enzyme used herein was Prime Star Max (manufactured by Dakara Bio Inc.). It was confirmed through 1% agarose electrophoresis that the obtained PCR product was a single band.

The purification of the PCR product was performed using a PCR purification kit (manufactured by Qiagen). The method was carried out in accordance with the method described in the manual. In order to check the purification degree and whether the PCR reaction was sufficiently performed, the purity and the amount of the DNA were measured using e-spect. It was determined that there was no problem since the measured purification degree was A₂₆₀ₙₘ/A₂₈₀ₙₘ = 1.8 or greater.

Next, the purified substance was used as a template and a cycle sequence was performed using a BigDye Terminator v3.1 Cycle Sequencing kit (manufactured by Applied Biosystems). The manual was referred to for the conditions. The base sequence of the obtained reactant was decoded using ABI PRISM 3100-Avant Genetic Analyzer (manufactured by Applied Biosystems).

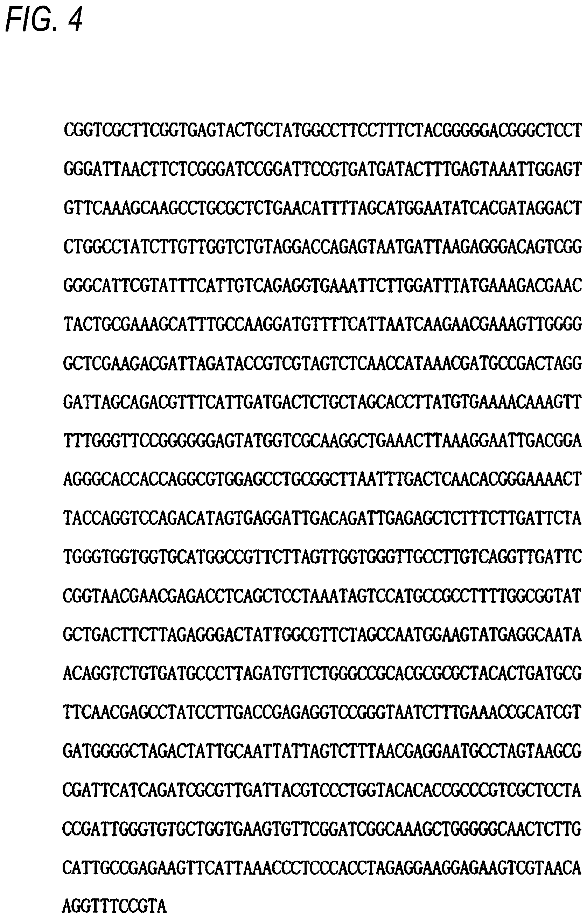

Homology analysis was performed using BLAST (Basic Local Alignment Search Tool). The method thereof was as follows. BLAST searching for the above-described sequence was conducted on the whole base sequence information in the data of the National Center for Biotechnology Information (NCBI). A species of an organism having a highest homology was regarded as a closely related species of the AVFF007 strains. Only the base sequence (1111 base, SEQ ID No: 1) which was set as a comparison target is shown in Fig. 4. Specifically, several bases at both the ends of the decoded base sequence were not set as a comparison target for the BLAST analysis, and thus are not shown in Fig. 4. The upper left of the base sequence shown in Fig. 4 is the 5'-terminal and the lower right thereof is the 3'-terminal.

As a result of the homology analysis, the above-described sequence had the homology (that is, 99% homology) to a 1109 base on the AVFF007 strain side among a Botryococcus sp. UTEX2629 strain side and 1118 bases on the Botryococcus sp. UTEX2629 strain side. Accordingly, the AVFF007 strains were classified as microalgae closely related to the Botryococcus sp. UTEX2629 strain.

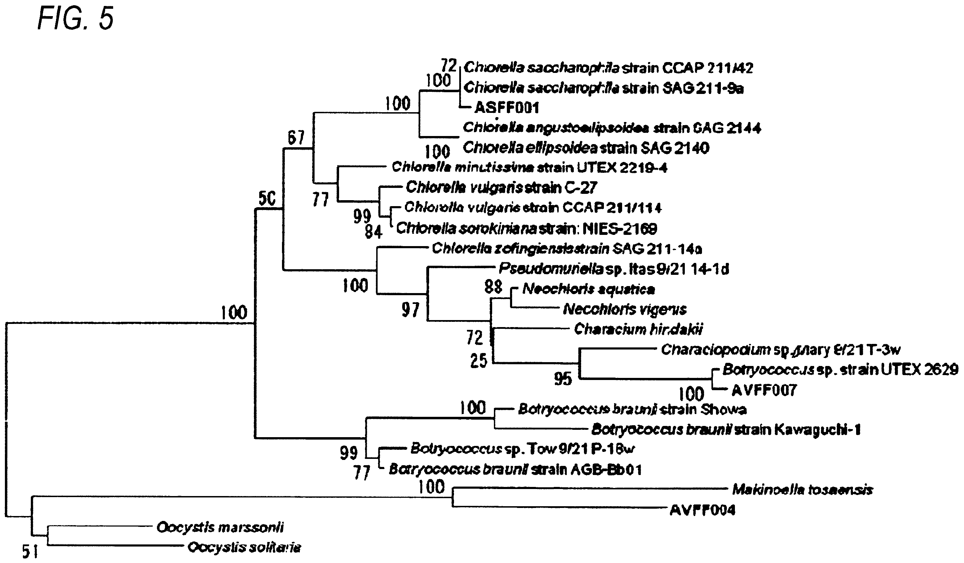

The schematic diagram obtained from the results of the above-described analysis is shown in Fig. 5. The AVFF007 strains are microorganisms which are also closely related to Characiopodium sp. Mary 9/21 T-3w, and there is a possibility that the name of the AVFF007 strains may be changed to the genus Characiopodium. In a case where the name of the Botryococcus sudeticus is changed, similarly, it is regarded in the present invention that the name of the AVFF007 strains is also changed. In addition, the same measure applies to a case where the name of the AVFF007 strain is changed to other names apart from the genus Characiopodium.

### (Density Measurement of AVFF007 Strains of Microalgae)

Cesium chloride was dissolved in a KOH solution (pH: 7.5) of 10 mM ethylenediaminetetraacetic acid (EDTA, ethylenediamine-N,N,N',N'-tetraacetic acid) and 5 mM HEPES (4-(2-hydroxyethyl)-1-piperazineethanesulfonic acid) to prepare solutions with a cesium chloride concentration of 35% to 105% (w/v) in steps of 10% in a cesium chloride concentration. Then, a concentration gradient was prepared such that the concentration becomes lower from a tip end portion of a Pollyallomer tube (manufactured by Hitachi Koki Co., Ltd.) toward a liquid surface portion thereof.

5 x 10⁶ cells/mL of AVFF007 strains were applied on the upper surface of the tube and centrifugal treatment was performed using a centrifuge for 30 minutes at a centrifugal force of 20000 x g at 4°C.

The cell density of algal bodies floating on the liquid surface was 1.26 g/mL, which is less than 1.34 g/mL which is the cell density of the Botryococcus sp. UTEX2629 strains disclosed in NPL 3.

From the above, it was determined that the algal bodies are not completely the same as the Botryococcus sp. UTEX2629 strains in view of the density and are microalgae close to the Botryococcus sp. UTEX2629 strains.

Furthermore, the fact that the algal bodies are microalgae closely related to the Botryococcus sp. UTEX2629 strains and they have almost the same form as each other can also be understood by comparing Fig. 3 of NPL 3 and the view showing the microphotograph of the AVFF007 strains shown in Fig. 3 of the present invention.

According to the culturing method of the present disclosure, it is considered that it is possible to form a biofilm on a liquid surface even with microalgae other than the above-described AVFF007 strains by setting favorable culture conditions.

Examples of the microalgae include ASFF001 strains, AVFF004 strains, and NIES2249 strains to be described below. The ASFF001 strains and the NIES2249 strains are preferable in view of the ability of forming a biofilm.

### (Microorganism Purification of ASFF001 Strains of Microalgae)

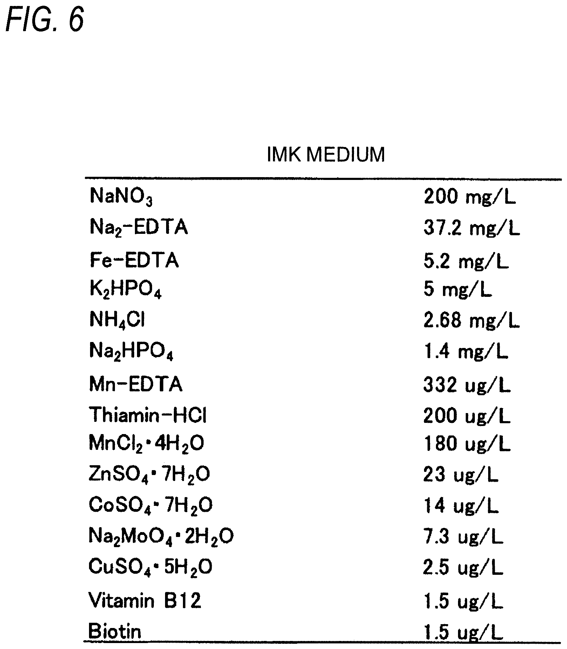

A biofilm which was adhered to the top of rock in a foot bath hot spring in Shizuoka-ken was stripped off and was collected by being put into a tube for homogenizing (TM-655S manufactured by Tomy Seiko Co., Ltd.) with a 5 mL capacity. Microorganism purification was performed by the same method as that for the AVFF007 strains. A mixture in which an IMK medium shown in Fig. 6 and a CSi medium shown in Fig. 2 were mixed at 1:1 (volume ratio) was used as a medium. The IMK medium was a mixture with artificial seawater, and Marine Art SF-1 (Tomita Pharmaceutical Co., Ltd.) was used as artifitial seawater. In addition, the IMK medium is different from a general IMK medium, and in the present invention, the IMK medium does not contain selenium.

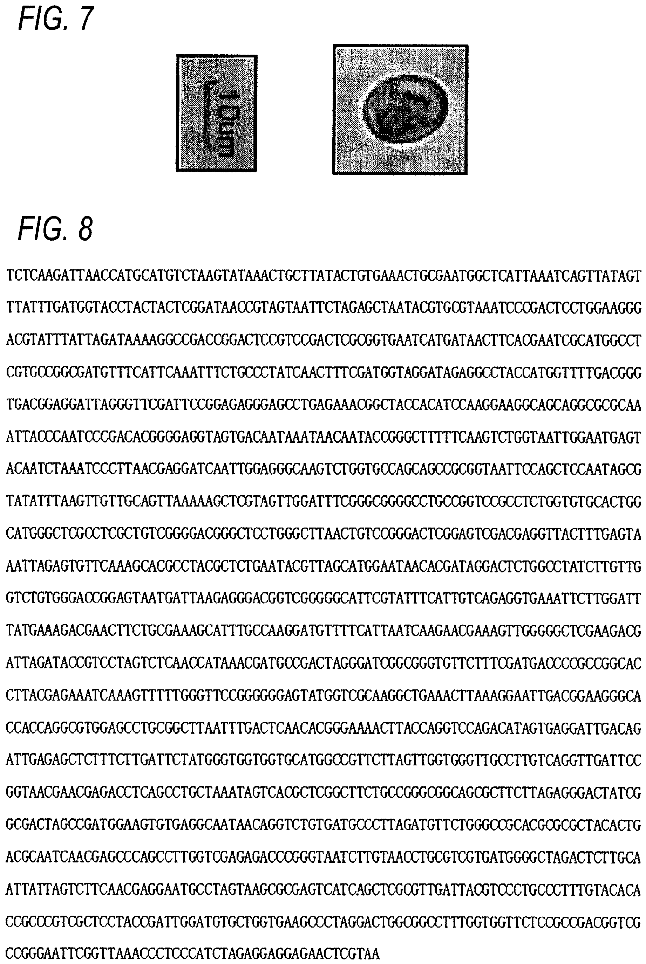

In addition, a view showing a microphotograph of ASFF001 strains at magnification of 40 is shown in Fig. 7. The properties of the ASFF001 strains are described below.
- There is homology to a 1725 base among Chlorella saccharophila and 1730 bases (Only the base sequence (1727 base, SEQ ID No: 2) which was set as a comparison target in the BLAST analysis is shown in Fig. 8. Specifically, several bases at both the ends of the decoded base sequence were not set as a comparison target for the BLAST analysis, and thus are not shown in Fig. 8. The upper left of the base sequence shown in Fig. 8 is the 5'-terminal and the lower right thereof is the 3'-terminal).
- It is possible to perform photoautotrophic culture through photosynthesis. The form of the strains is an elliptical shape.
- Green algae
- The strains have a size 5 µm to 20 µm and exist as a mixture in various sizes.
- The strains show a property of floating on a liquid surface.

### (Microorganism Purification of AVFF004 strains of Microalgae)

5 mL of natural fresh water from a pond in Kyoto was collected by being put into a tube for homogenizing (TM-655S manufactured by Tomy Seiko Co., Ltd.). Microorganism purification was performed by the same method as that for the AVFF007 strains. A mixture in which a C medium and a CSi medium were mixed at 1:1 (volume ratio) was used as a medium.

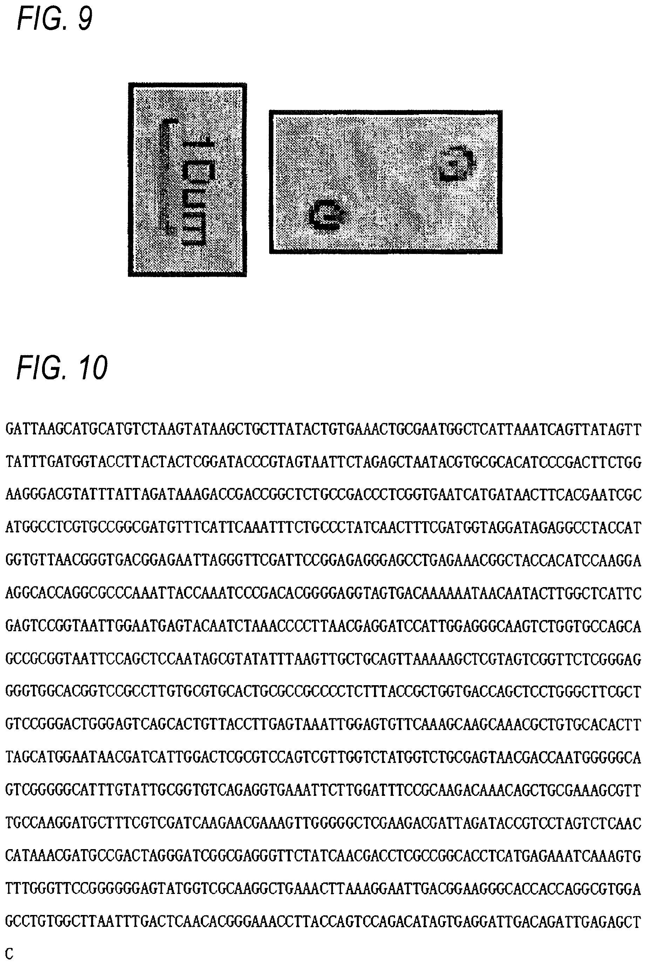

In addition, a view showing a microphotograph of AVFF004 strains at magnification of 40 is shown in Fig. 9. The properties of the AVFF004 strains are described below.
- There is homology to a 1144 base among Makinoella tosaensis and 1215 bases (Only the base sequence (1201 base, SEQ ID No: 3) which was set as a comparison target in the BLAST analysis is shown in Fig. 10. Specifically, several bases at both the ends of the decoded base sequence were not set as a comparison target for the BLAST analysis, and thus are not shown in Fig. 10. The upper left of the base sequence shown in Fig. 10 is the 5'-terminal and the lower right thereof is the 3'-terminal).
- It is possible to perform photoautotrophic culture through photosynthesis. The form of the strains is elliptical or droplet shapes.
- Green algae
- The strains have a size 1 µm to 5 µm.
- The strains show a property of floating on a liquid surface.

The AVFF004 strains as microalgae used in Examples of the present specification are internationally deposited at the Patent Organism Depositary of the National Institute of Technology and Evaluation (Room No. 120, 2-5-8 Kazusakamatari Kisarazu-shi, Chiba-ken, Japan) dated December 21, 2011 with a deposition number of FERM BP-11444 [or at the Patent Microorganism Depository of the National Institute of Advanced Industrial Science and Technology during international deposition (Central 6, 1-1, Higashi 1-chome Tsukuba-shi, Ibaraki-ken, Japan)] under the Budapest Treaty.

The NIES-2249 strains are Chlorococcum echinozygotum Starr purchased from the National Institute of Environmental Studies.

### [Method of the Present Invention]

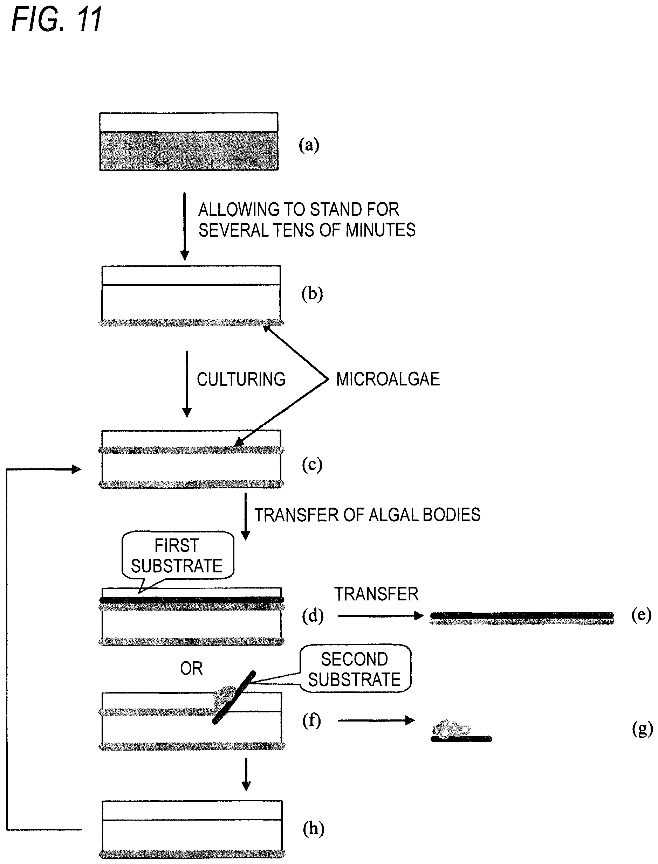

A basic structure of the culturing method in accordance with the present invention that is defined in the claims is shown in Fig. 11. The schematic view is for describing the present invention, and therefore, description thereof is simplified in some sections.

As shown in (a) of Fig. 11, first, a suspended solution or a dispersed solution of microalgae is prepared. Next, when the culture container is set in a stationary state, the microalgae generally sink to the bottom surface in several seconds to several tens of minutes depending on the kinds of microalgae as shown in (b) of Fig. 11. When the microalgae are cultured in this state for a while, a biofilm formed of the microalgae is formed on the liquid surface as shown in (c) of Fig. 11. Generally, microalgae exist on the bottom surface of the culture container as shown in (c) of Fig. 11, and also exist on the side surface of the culture container although this is not shown in the drawing.

In the present invention, it is preferable that the culture container be in a stationary state during the culturing. However, it is possible to perform the same culturing as that of the present invention even when shaking culture is performed as long as the container is not strongly stirred. However, it is preferable to perform the culturing in a stationary state since the number of algal bodies in the biofilm formed on the liquid surface increases. In addition, the sinking of the microalgae to the bottom surface of the culture container means that the majority of microalgae sink to the bottom surface thereof and does not mean a state in which the microalgae completely disappear from the top of the liquid surface or the middle of the liquid. For example, when shaking culture is performed, there are 1/6 times as many floating algal bodies on the liquid surface compared to the case where the stationary culture is performed, the number of algal bodies in the middle of the medium become 3.5 times that of the stationary culture, and the number of algal bodies on the bottom surface become 1.4 times that of the stationary culture. That is, the proportion of algal bodies existing on the liquid surface is decreased and the proportion of algal bodies existing in the middle of the medium and on the bottom surface is increased by the shaking culture.

When the biofilm formed on the liquid surface is brought into contact with a first substrate as shown in (d) of Fig. 11, the biofilm adheres to the surface of the first substrate ((e) of Fig. 11). The adhered biofilm (mainly algal bodies) can be collected by being peeled off from the first substrate (hereinafter, also referred to as "collection through transfer"). In the drawing, the substrate is brought into contact with the whole liquid surface of the culture container. However, the substrate may be brought into contact a part of the liquid surface, or whole contact and part contact may be repeated plural times. The collection efficiency of the microalgae on the liquid surface is improved by performing contact plural times in this manner. It is possible to transfer a film-like structure or a three-dimensional structure so as to be superposed on the surface of the first substrate. It is preferable to perform the transfer once in a case of a culture area less than 1 m², and to perform the transfer several times in a case of a culture area greater than or equal to 1 m². The details of the film-like structure and the three-dimensional structure are as described below.

Hereinafter, the collection by the transfer will be described in more detail.

### [Collection of Biofilm on Liquid Surface Through Transfer]

First, a transfer material to which the biofilm on the liquid surface is adhered is prepared as a first substrate. Here, the first substrate is a substrate which is used for transferring the film-like structure or the three-dimensional structure formed of microalgae on the liquid surface and is used in (d) of Fig. 11 for collection. A substrate covering the whole surface of the culture container is used as the first substrate in the drawing. However, the substrate covering the whole surface of the culture container may be used in this manner and a substrate which can cover only a portion of the culture container may be used as the first substrate.

Glass, polyethylene, polypropylene, nylon, polystyrene, vinyl chloride, polyester, and the like can be used as the transfer materials, but the transfer materials are not limited thereto. It is desirable that the transfer material can be cut using scissors or the like as necessary so as to make the size of the transfer material smaller than that of the area of the culture container. For example, it is preferable to cut the transfer material to have a circular shape with a diameter of 3.5 cm in a case in which the culture container is a 6 hole plate. It is preferable to wash the cut transfer material in order to remove debris on the surface. When using the collected biofilm for the next culturing, it is preferable to use it after additionally immersing it in ethanol for disinfection and drying the surface of the transfer material.

Next, the cut transfer material is gently inserted so as to be parallel to or almost parallel to the liquid surface formed by the culture container, and the microalgae on the liquid surface are adhered to the cut transfer material. When performing the insertion, the cut transfer material is slightly obliquely inserted with respect to the liquid surface, and the transfer material is finally made parallel to the liquid surface. Then, it is possible to collect a large amount of biofilm on the liquid surface with a smaller number of times of transfer, which is preferable. It is possible to transfer the biofilm from the liquid surface of the culture container to the cut transfer material by gently lifting the cut transfer material to which the biofilm on the liquid surface is adhered. The transfer of the biofilm on the liquid surface using the first substrate may be performed several times. This is because the transfer rate is improved by performing the transfer several times.

Any methods may be used as the method for collecting the biofilm from the first substrate (for example, the cut transfer material) as long as it is possible to peel off the microalgae from the top of the substrate. It is possible to peel off the biofilm from the transfer material by, for example, applying a stream of water; subjecting the container, into which the cut transfer material is put, to an ultrasonic treatment; and strongly shaking or performing a high speed-shaking treatment after closing the container, into which the cut transfer material is put, with a lid. Among these, a method for peeling of the biofilm from the substrate using an instrument such as a cell scraper for which a material that does not damage the first substrate is used is preferable. In addition, the first substrate may be reused many times.

In some cases, the biofilm of microalgae proliferating on the liquid surface in the culture container grows from a film shape to a pleated shape in the culture medium depending on the state of culturing. In this case, it is possible to collect the pleated biofilm using a pipette.

Next, another collecting method other than that by the transfer will be described.

As shown in (f) of Fig. 11, it is possible to scrape the biofilm on the liquid surface using a second substrate for collection. Here, the second substrate is a substrate which is used for collecting a film-like structure or a three-dimensional structure formed of microalgae on the liquid surface and is used in (f) of Fig. 11.

In the drawing, the substrate is moved from the left side to the right side. The movement direction of the second substrate may be reversed (that is, the movement of the substrate from the left side to the right side of the drawing) or the collection may be performed several times. The collection rate is improved by performing the collection several times. When performing the collection several times, the second substrate may be used in a state in which the biofilm is adhered thereto; a substrate after entirely or partially removing the biofilm from the surface of the above-described first substrate may be used as the second substrate; or a new substrate may be used. In addition, only a sheet of the second substrate is drawn in Fig. 11, but plural sheets of the second substrate may be used at the same time. By doing this, the collection rate improves. Among these, resuming the collection using the same second substrate after removing the collected biofilm using a sheet of the second substrate is preferable as much as the strength of the second substrate allows in terms of installation cost of the collection device or the like. In addition, it is possible to freely set the size of the second substrate, and the angle, the movement speed, or the like of the second substrate with respect to the liquid surface depending on the purpose, (g) of Fig. 11 is a state in which the biofilm is collected on the second substrate.

Hereinafter, the collection using the second substrate will be described in more detail.

### [Collection of Biofilm on Liquid Surface Using Second Substrate]

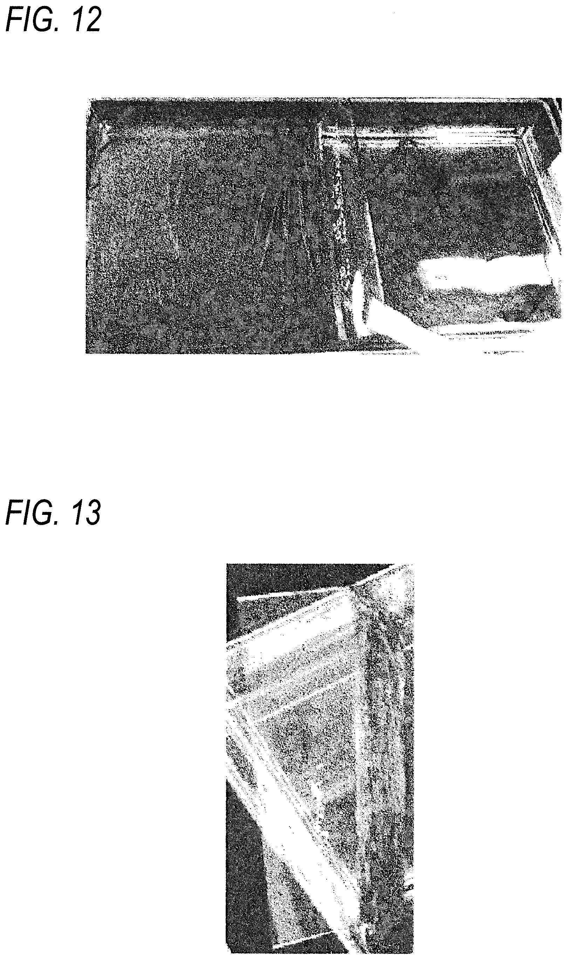

Fig. 12 is a state in which a film-like structure formed of microalgae floating on a liquid surface is collected using the second substrate (slide glass (76 mm x 26 mm) after forming a biofilm (film-like structure in the example of the drawing) on the liquid surface.

In Fig. 12, the biofilm is collected by obliquely inserting the long side of the slide glass into the biofilm on the liquid surface and advancing the slide glass in a left direction as it is, and by depositing the biofilm on the liquid surface on the slide glass. The left side of the drawing indicates a liquid surface on which the biofilm before being collected is formed and the right side of the drawing indicates a liquid surface after the biofilm is collected.

Fig. 13 is a state of a biofilm deposited so as to be folded over the slide glass. The collection using the second substrate corresponds to processes from (f) to (g) of Fig. 11. In addition, it is possible to use substrates such as a nylon film other than a slide glass as the second substrate. In addition, the size of the second substrate can be appropriately changed depending on the size of the culture container. However, it is preferable to use a substrate smaller than that of the surface area of the culture container as the second substrate.

In regards to the timing of collecting the biofilm on the liquid surface, it is possible to collect the biofilm in a state in which the liquid surface in the culture container is partially covered with the biofilm. However, it is preferable to collect the biofilm when the liquid surface in the culture container is completely covered with the biofilm in view of obtaining a large number of algal bodies of the microalgae. In addition, the collection may be performed after continuing culture for a while after the liquid surface is completely covered with the biofilm.

Particularly, it is preferable to collect the biofilm after a three-dimensional structure, to be described later, is formed on the liquid surface. In general, the three-dimensional structure is a structure which can be seen when the proliferation of the microalgae in a film-like structure is further progressed. In the three-dimensional structure, a larger quantity of microalgae which can be collected and a lower water content ratio can be obtained compared to the two-dimensional film-like structure.

(h) of Fig. 11 is a state after the biofilm on the liquid surface is collected. Microalgae are adhered to or deposited on the bottom surface of the culture container. In the schematic view of the present invention, there is description that the supply of the microalgae is performed from the bottom surface to the top of the liquid surface. However, in reality, there are microalgae at a low concentration in the medium even at other than the liquid surface and the bottom surface. Even in the state where the microalgae are supplied from the liquid surface and the bottom surface to the middle of liquid, in the present invention, it is described such that the microalgae are supplied from the bottom surface of the culture container to the top of the liquid surface. In addition, there are two cases for the supply of the microalgae from the bottom surface of the culture container to the top of the liquid surface, including a case in which the microalgae are moved to the top of the liquid surface without actually accompanying proliferation of the microalgae on the bottom surface and a case in which the microalgae proliferate while being moved from the bottom surface to the top of the liquid surface.

The culture container may be an open system or a closed system. However, it is more preferable that the culturing be performed in a closed system in view of prevention of microorganisms other than target microorganisms, or debris from being mixed thereinto from the outside, prevention of algae on the liquid surface from being destroyed due to wind, prevention of algae on the liquid surface from being dried, and prevention of water from being evaporated.

In addition, only the biofilm on the liquid surface may be collected or both the microalgae in the biofilm on the liquid surface and on the bottom surface may be collected. This is because it is possible to use both the microalgae on the liquid surface and the bottom surface as a biomass.

In addition, in some cases, the biofilm of the microalgae proliferating on the liquid surface in the culture container grows from a film shape to a pleated shape in the culture medium depending on the state of culturing. In this case, it is possible to collect the pleated biofilm by making the depth of inserting the second substrate into the liquid deep.

In the present invention, as shown in the state of (c) of Fig. 11, the culturing method for culturing the microalgae on the liquid surface is called liquid surface-floating culture. That is, a culturing method for culturing the microalgae in only any one of the middle of the liquid or the bottom surface of the liquid, or in both the middle of the liquid and the bottom surface of the liquid is not included in the liquid surface-floating culture.

The liquid surface in the present invention is typically a liquid surface of a liquid medium to be described later, and is generally an interface between the liquid medium and the air.

Furthermore, performing the liquid surface-floating culture in a stationary state as in (c) of Fig. 11 is called liquid surface-floating culture through stationary culture in the present invention.

Any method for collecting the biofilm from the second substrate may be used as long as it is possible to peel off the biofilm from the top of the substrate. However, it is preferable to use an instrument such as a cell scraper for which a material that does not damage the second substrate is used. In addition, the second substrate may be reused many times.

According to an example of the embodiments of the present invention, it is possible to prepare a suspended solution or a dispersed solution of microalgae by dispersing microalgae which are obtained through a microorganism purification process in a liquid medium containing an artificial medium. In addition, it is possible to form a film-like structure or a three-dimensional structure of microalgae on a liquid surface of a liquid medium and to collect proliferating microalgae from the entire culture tank or the top of the liquid surface by performing the culture in a culture apparatus.

In the above-described collecting method, it is preferable that greater than or equal to 70% of the biofilm formed on the liquid surface be collected. It is more preferable that greater than or equal to 80% thereof be collected. It is still more preferable that greater than or equal to 90% thereof be collected. It is particularly preferable that 100% thereof be collected. It is possible to visually check the collection rate of the biofilm formed on the liquid surface, for example.

### [Microorganism Purification Process]

The microorganism purification process referred to in the present invention is a process of isolating only target microalgae from a state in which various kinds of organisms, debris, and the like coexist together with microalgae in a case of microalgae in a natural state. Such complete isolation is practically difficult, and therefore, in the present invention, it is defined that microorganism purification is completed as long as the target microalgae are primarily obtained. The primary acquisition thereof in the present invention means a case in which microalgae other than a target could not be confirmed in several visual fields when observed with a microscope.

Examples of the microorganism purification method include a method for collecting a colony which is formed through culturing after developing a diluted suspended solution containing microalgae on an agar medium, or a method for collecting microalgae one by one under a microscope.

### [Pre-culturing Process]

The pre-culturing process referred to herein is a process of increasing the number of microalgae until the primary culturing can be performed by causing stored microalgae to proliferate while microalgae obtained after the completion of the microorganism purification process is generally stored. Any well-known method can be used as the culturing method in the pre-culturing process. In addition, it is possible to perform the liquid surface-floating culture of the present invention. In addition, the pre-culturing process may be performed several times to cause the microalgae to proliferate until the microalgae reach a scale in which the primary culturing can be performed.

In addition, a culturing tank having a surface area of 1 cm² to 1 m² is generally used. It is possible to perform the culturing in either of an indoor place and an outdoor place, but it is preferable to perform the culturing indoors.

### [Primary Culturing Process]

The primary culturing process referred to herein is a culturing process which is performed after the pre-culturing process is performed and immediately before the final collecting process is performed. The primary culturing process may be performed several times.

In addition, a culturing tank having a surface area greater than or equal to 100 cm² is generally used. It is possible to perform the culturing in any of an indoor place and an outdoor place, but it is preferable to perform the culturing outdoors.

### [Use of Microalgae]

Microalgae, which were obtained through any well-known culturing method such as floating culture, liquid surface-floating culture, and adherent culture, may be used as microalgae used for performing the pre-culturing and the primary culturing. However, it is preferable to perform suspension treatment in the liquid surface-floating culture.

### [Suspension Treatment]

In the present invention, any treatment method for making aggregates of microalgae be smaller aggregates or single microalgae can be included in the suspension treatment. Examples thereof include a gentle treatment such as a treatment of pipetting or shaking a solution of microalgae in a container by hand and a treatment using a stirrer chip or a stirring rod; a strong treatment such as an ultrasonic treatment or high speed-shaking treatment; and a method using a substance such as an enzyme decomposing an adhesion substance such as a matrix between cells.

The contact area between the microalgae and the medium increases as the size of the aggregates of the microalgae become smaller compared to the aggregates of the microalgae having a larger size, when making the microalgae be smaller aggregates or single microalgae by the suspension treatment. As a result, in some cases, it is possible to improve the proliferation rate of the microalgae, which is preferable.

The ultrasonic treatment is a method of directly applying oscillatory waves, which have high oscillation frequency and cannot be heard by the human ear, to a solution of microalgae or a container holding the solution of microalgae, and is a method in which the container is not necessarily a closed container and the solution containing the microalgae is not separated from the bottom surface of the container.

The high speed-shaking treatment refers to a treatment method in which a solution containing an aggregate of microalgae is added to a closed container for shaking such that an air layer can be formed and the entire closed container for shaking is shaken at a high speed. In the high speed-shaking treatment, the suspended solution layer is separated from or brought into contact with the inner wall of the closed container for shaking.

### [Suspended solution]

The suspended solution referred to in the present invention is a solution on which a suspension treatment is performed.

### [Suspended solution of Microalgae]

The suspended solution of microalgae refers to a solution which is obtained by performing a suspension treatment on microalgae. Specifically, the suspended solution of microalgae is the solution used in (a) of Fig. 11. Microalgae which are cultured while floating in liquid may be used or microalgae which are cultured while adhering on the substrate may be used after being peeled off from the surface of the substrate. In addition, microalgae which are obtained by the liquid surface-floating culture may be used. In addition, a mixture of microalgae derived from at least two or more of the culturing forms may be used. Examples thereof include a mixture of microalgae derived from the liquid surface-floating culture and microalgae on the bottom surface of the culture container during the liquid surface culture.

By conducting the suspension treatment, it is possible to make the microalgae be smaller aggregates of microalgae or single microalgae, to make the microalgae be uniformly deposited on the bottom surface of the culture container, and to efficiently use the surfaces. Moreover, light or nutrients required for the proliferation of the microalgae can be easily obtained. Accordingly, it is considered that the proliferation rate of the microalgae improves, and as a result, the proliferation rate of the liquid surface-floating culture also improves. Furthermore, when the suspension treatment is performed, the film structure of the film-like structure formed of microalgae on a liquid surface becomes uniform, thereby reducing unevenness of the surface. Therefore, the transfer to the first substrate becomes easy and its efficiency also improves.

In addition, the suspension treatment may be performed for the pre-culturing and the primary culturing in order to check the proliferation state of the microalgae. This is because it becomes easy to count the number of microalgae by this treatment.

### [Dispersion Treatment]

Herein, the dispersion treatment is a treatment method for making aggregates of microalgae be smaller aggregates or single microalgae. For example, the dispersion treatment refers to a suspension treatment other than a gentle treatment such as a treatment of pipetting or shaking a solution of microalgae in a container by hand and a treatment using a stirrer chip or a stirring rod. That is, the dispersion treatment includes a strong treatment such as an ultrasonic treatment or high speed-shaking treatment and a method using a substance such as an enzyme decomposing an adhesion substance such as a matrix between cells.

### [Dispersed solution]

The dispersed solution referred to herein is a solution in which a dispersion treatment such as an ultrasonic treatment or high speed-shaking treatment is performed, and is not a solution in which a gentle suspension treatment such as a treatment of pipetting or shaking a solution of microalgae in a container by hand and a treatment using a stirrer chip or a stirring rod is performed.

### [Dispersed solution of Microalgae]

The dispersed solution of microalgae refers to a solution which is obtained by performing dispersion-treatment on the microalgae. Specifically, the suspended solution of microalgae is the solution used in (a) of Fig. 11. Microalgae which are cultured while floating in liquid may be used, microalgae which are cultured while adhering on the substrate may be used after being peeled off from the surface of the substrate, or microalgae which are peeled off from the surface of the substrate at the same time as the suspension treatment may be used. In addition, the microalgae which are obtained by the liquid surface-floating culture may be used. In addition, a mixture of microalgae derived from at least two or more of the culturing forms may be used. Examples thereof include a mixture of microalgae derived from the liquid surface-floating culture and microalgae on the bottom surface of the culture container during the liquid surface culture.

By conducting the dispersion treatment, it is possible to make the microalgae be smaller aggregates of microalgae or single microalgae, to make the microalgae be uniformly deposited on the bottom surface of the culture container, and to efficiently use the surfaces. Moreover, light or nutrients required for the proliferation of the microalgae can be easily obtained. Accordingly, it is considered that the proliferation rate of the microalgae improves, and as a result, the proliferation rate of the liquid surface-floating culture also improves. Furthermore, when the suspension treatment is performed, the film structure of the film-like structure formed of microalgae on a liquid surface becomes uniform, thereby reducing unevenness of the surface. Therefore, the transfer to the first substrate becomes easy and its efficiency also improves.

In addition, the dispersion treatment may be performed for the pre-culturing and the primary culturing in order to check the proliferation state of the microalgae. This is because it becomes easy to count the number of microalgae by this treatment.

### [Deposition]

The deposition referred to herein refers to a state in which microalgae exist in or adhered to the vicinity of the bottom surface of the culture container or a state in which both the states are mixed. The state in which microalgae exist in the vicinity thereof refers to a state in which the microalgae are easily moved from the substrate or the bottom surface of the culture container by slight movement of a liquid medium. In addition, the deposition includes a state of being adhered. It is desirable that the microalgae be deposited once as shown in Fig. 11. This is because the film-like structure which is formed of microalgae and is formed on the liquid surface through proliferation becomes uniform by performing the deposition, thereby improving the efficiency of the transfer to the first substrate.

### [Adhesion]

The adhesion referred to herein refers to a state in which microalgae are directly adhered to the substrate or the bottom surface of the culture container and in which microalgae are adhered thereto to the extent that the microalgae are not peeled off from the substrate or the bottom surface of the culture container by slight movement of a liquid medium. In addition, in some cases, a state in which a biofilm (film-like structure or three-dimensional structure), which is formed of microalgae and is formed on a liquid surface of a liquid medium, floats on a liquid surface is described as a state in which the biofilm is adhered to the liquid surface.

### [Deposition Process]

The process of depositing microalgae as shown in (a) to (b) of Fig. 11 is a process of depositing microalgae on the bottom surface of a culture container by allowing a suspended solution or a dispersed solution of microalgae to stand. As shown in (c) of Fig. 11, there is a possibility that the microalgae may be precipitated on the surface of the bottom surface of the culture container from a biofilm after the biofilm (film-like structure or three-dimensional structure) formed of microalgae is partially formed on a liquid surface. However, preferably, such a case is not included in the deposition process. In general, it takes several days to about two weeks until at least a biofilm formed of microalgae on the liquid surface can be visually confirmed after the deposition while this depends on the kind of microalgae. However, such a process is not referred to as the deposition process and is included in the culturing process. The deposition process can progress even in a shaking state. However, it is preferable to set the state to be stationary because the number of deposited microalgae increases in the stationary state. In addition, in the present invention, almost all of microalgae may be deposited when visually checked, but when observed using an instrument such as a microscope, there is an extremely small quantity of microalgae contained in the top of the liquid surface or in the middle of the liquid. However, herein,
even in such a case, this is described as being deposited since most of the microalgae sink to the bottom surface.

In the deposition process, it takes 1 second to 1 day for the microalgae to be precipitated on the surface of the bottom surface of the culture container depending on the kind thereof. In a case where the deposition process accompanies adhesion, a longer time is taken and the deposition process overlaps with the culturing process in some cases.

### [Culturing Process]

The culturing process refers to a process immediately before the biofilm (film-like structure or three-dimensional structure) formed of the microalgae on the liquid surface is collected from the process after the microalgae are deposited. That is, the culturing process refers to the process from (b) to (c) of Fig. 11 or the process from (h) to (c) of Fig. 11.

The culturing process is a process in which at least a portion of deposited microalgae rises to the top of the liquid surface and a biofilm formed of microalgae is formed on a liquid surface. In order to form the biofilm formed of microalgae, the microalgae needs to proliferate. It is considered that there is a case in which the deposited microalgae are supplied to the top of the liquid surface after proliferating or a case in which the microalgae floating on the liquid surface proliferate on the liquid surface, or a combination of both the cases.

The culturing process also includes a process in which a biofilm is formed on the liquid surface, and then, the structure further proliferates. In general, a three-dimensional structure is formed after a film-like structure is formed, and the process is also included in the culturing process.

In the culturing process disclosed herein, there is little odor accompanying the culturing.

Shaking culture may be performed in the culturing process. This is because it is possible to form the film-like structure or the three-dimensional structure formed of microalgae on a liquid surface even through the shaking culture although the number of microalgae is smaller than that obtained by the stationary culture. However, it is preferable to perform the stationary culture in order to increase the proliferation number of biofilms formed of the microalgae on the liquid surface. In addition, the stationary culture does not require any power for shaking, stirring, or the like, and does not require any power energy or a power generator unlike the floating culture in the related art, thereby reducing the cost greatly, which is preferable.

That is, in the method for culturing the microalgae of the present invention, it is preferable to include the stationary culture of the microalgae capable of forming the biofilm on the liquid surface due to the above reasons.

### [Distribution State of Microalgae]

In the culturing of the microalgae using the culturing method of the present invention, the distribution state of microalgae immediately before the collection of the biofilm may be performed by the transfer using the first substrate, or using the second substrate has a characteristic in that the number of microalgae in the middle of liquid is smaller than the number of microalgae on the liquid surface or the number of microalgae deposited on the bottom surface of the culture container. In addition, in the distribution state at this time, it is preferable that the number of microalgae on the liquid surface be larger than that deposited on the bottom surface of the culture container. However, the present invention, as defined in the claims, is not limited thereto. The number of microalgae referred to herein does not include zoospores which are generated from cells of the microalgae and which are difficult to observe using a microscope.

The number of microalgae in the middle of the liquid refers to the number of microalgae which is counted in the vicinity of the midpoint between the liquid surface and the bottom surface of the culture container, and refers to the number of microalgae per square centimeter. In addition, the number of microalgae in the vicinity of the top of the liquid surface or the bottom surface of the culture container refers to the number of microalgae per cubic centimeter. It is regarded that the number of microalgae can be replaced using a method which can determine the quantity of microalgae such as the weight, the dry weight, and the turbidity of microalgae.

### [Stationary Culture]

The stationary culture referred to herein is a culturing method of culturing microalgae in a state of the microalgae not being intentionally moved. That is, for example, in some cases, a medium is circulated in accordance with local change in the medium temperature and microalgae are moved by the flow. However, such a case is also called stationary culture since the movement of the microalgae is not intentionally caused.

In the present invention, it is preferable to perform culture in the stationary state (that is, perform stationary culture) in all processes. This is done in order to prevent the biofilm (film-like structure or three-dimensional structure) formed of microalgae on a liquid surface from being destroyed. This is because there is a possibility that the collection efficiency using the second substrate may deteriorate and the microalgae may move to the middle of liquid or to the bottom surface of the culture container when the biofilm formed of microalgae is destroyed. However, even if the culturing state is not stationary, it is possible to form the biofilm formed of microalgae on the liquid surface even when the number of microalgae is small. Therefore, the culturing in the stationary state is preferable in the present invention, but there is no restriction.

Herein, examples of methods of creating a state which is not stationary include a method of shaking the entire culture container, a method of performing stirring in the culture container using a stirring bar or a stirring rod such as a stirrer chip, a method of bubbling gas containing air or high-concentration carbon dioxide, and a method of making a suspended liquid of microalgae flow.

### [Adherent Culture]

The adherent culture referred to herein is culturing in a state in which microalgae are adhered on the surface of a substrate or the wall surface of a culture container (for example, the bottom surface or the side surface of the culture container).

Microalgae may be temporarily adhered on the substrate using a medium formed of a different composition in order to promote the adhesion of the microalgae. For example, it is possible to use a medium to which calcium that promotes the adhesion of the microalgae is added. The information that calcium participates in adhesion of microalgae, in particular, diatoms is disclosed in Plant. Physiol. (1980) 65, 129-131.

### [Floating Culture]

Herein, the culturing of microalgae in liquid (in particular, in a liquid medium to be described later) is referred to as floating culture.

A state in which microalgae are cultured while floating in liquid other than at the liquid surface, the side surface or the bottom surface of the culture container is called floating culture.

### [Liquid Surface-floating Culture]

Herein, the culturing method for culturing microalgae on a liquid surface is called liquid surface-floating culture.

In addition, even when microalgae exist on the bottom surface of a culture container and in the middle of a medium at the same time, the culturing method is also called liquid surface-floating culture. Furthermore, microalgae being cultured by being adhered on the liquid surface can also be considered as liquid surface-floating culture, and thus, in the present invention, the liquid surface-floating culture is considered as a kind of adherent culture.

In addition, in some cases, a phenomenon in which aggregates of microalgae seep out of the biofilm (film-like structure or three-dimensional structure) on the liquid surface to the middle of the liquid can be seen when the liquid surface-floating culture is performed in the present invention. In the present disclosure, the culturing in such a state is included in liquid surface-floating culture.

It is possible to perform the liquid surface-floating culture and to perform any one of the above-described floating culture and the adherent culture or both the floating culture and the adherent culture at the same time.

As described above, the names of the culturing methods are distinguished as described above depending on the place of culturing microalgae in the liquid medium in the present disclosure, but the methods can be performed at the same time as described above. That is, a case, in which the microalgae are cultured on the liquid surface and cultured while being dispersed in the middle of the liquid at the same time, is called the liquid surface-floating culture and the floating culture being simultaneously performed.

For example, a well-known method that can culture microalgae belonging to the genus Botryococcus can be applied as the process of culturing microalgae of the present invention by the liquid surface-floating culture, the floating culture, and the adherent culture. For example, microalgae may be inoculated into a culture container such as conical flask into which a liquid medium is put, and be aeration-cultured under irradiation of light while being allowed to stand.

Next, the liquid medium which can be used in the liquid surface-floating culture, the floating culture, and the adherent culture will be described.

### [Medium (Liquid Medium)]

In the present invention, any well-known medium (liquid medium) can be used as long as it is possible to culture microalgae. It is desirable that the medium be selected depending on the kinds of microalgae to be cultured. For example, since the above-described AVFF007 strains are grown in fresh water, the media are preferably fresh water media. Examples of well-known media include an AF-6 medium, an Allen medium, a BBM medium, a C medium, a CA medium, a CAM medium, a CB medium, a CC medium, a CHU medium, a CSi medium, a CT medium, a CYT medium, a D medium, an ESM medium, an f/2 medium, a HUT medium, an M-11 medium, an MA medium, an MAF-6 medium, an MF medium, an MDM medium, an MG medium, an MGM medium, an MKM medium, an MNK medium, an MW medium, a P35 medium, a URO medium, a VT medium, a VTAC medium, a VTYT medium, a W medium, a WESM medium, a SW medium, and a SOT medium. Among these, fresh water media are an AF-6 medium, an Allen medium, a BBM medium, a C medium, a CA medium, a CAM medium, a CB medium, a CC medium, a CHU medium, a CSi medium, a CT medium, a CYT medium, a D medium, a HUT medium, an M-11 medium, an MA medium, an MAF-6 medium, an MDM medium, an MG medium, an MGM medium, an MW medium, a P35 medium, a URO medium, a VT medium, a VTAC medium, a VTYT medium, a W medium, an SW medium, and an SOT medium. As media for culturing the above-described AVFF007 strains, a C medium, a CSi medium, and a CHU medium, and a mixture of these media are preferable, and a C medium and a CSi medium, and a mixture of these media are more preferable.

The media may be subjected to ultraviolet ray sterilization, autoclave sterilization, and filter sterilization, or may not be sterilized.

In the present invention, it is preferable that the liquid medium contain calcium. That is, it is desirable to add calcium to the medium. This is because the proliferation speed of the microalgae improves, and thus it is easy to form a biofilm on a liquid surface (film-like structure or three-dimensional structure), when calcium is added to the medium. The concentration of calcium in the liquid medium is not particularly limited, but is preferably greater than or equal to 0.3 mM and more preferably greater than or equal to 0.5 mM. The upper limit value of the concentration of calcium in the liquid medium is not particularly limited, but is generally less than or equal to 100 mM, preferably less than or equal to 50 mM, and more preferably less than or equal to 5 mM.

### [Water Depth]

The water depth of a liquid medium used in the present invention is not particularly limited, but it is preferable that the water depth be shallow. This is because the shallower the water depth, the smaller the amount of water used. In addition, this is because complication during handling, such as movement of water, or the amount of energy used also decreases. Furthermore, this is because the manufacturing cost of culture containers decreases. The water depth is preferably greater than or equal to 0.4 cm, more preferably 1.0 cm to 10 cm, still more preferably 2.0 cm to 1 m, and particularly preferably 5.0 cm to 30 cm. When the water depth is greater than or equal to 0.4 cm, it is possible to form the biofilm. When the water depth is greater than or equal to 1.0 cm, the culturing of microalgae being in an unfavorable state caused by water evaporating while the biofilm is formed on the liquid surface is sufficiently avoided. When the water depth is less than or equal to 10 m, the handling of a suspended liquid containing a medium or microalgae being too difficult can be avoided and it is possible to suppress decrease in usage efficiency of light caused by the liquid absorbing light. When the water depth is 5.0 cm to 30 cm, a lower amount of water evaporates while the biofilm is formed, the handling of a suspended liquid containing a medium or microalgae becomes easy, and the usage efficiency of light improves since the absorption of light due to microalgae infinitesimally existing in water or a medium is minimized. In addition, it is easy to supply carbon dioxide and to release oxygen generated through photosynthesis to the air as the water depth is shallower.

According to the present invention, basically, the middle of the liquid mainly plays a role in supplying nutrients for culturing and plays a small role as a space used for growth or proliferation of microalgae. For this reason, it is possible to make the depth of a liquid medium shallow, and to thereby greatly reduce the liquid amount of the medium. Accordingly, the handling of movement of liquid becomes easy as well as the cost of water or the medium being greatly reduced. In addition, the treatment cost of the used medium becomes easy because of a lower amount of liquid medium. Furthermore, the shallow water depth is advantageous also for diffusion of carbon dioxide (CO₂) in a gas phase to the entire liquid medium tank. In addition, the deeper the water depth of a culture pond, the greater the amount of light absorbed by the medium. However, since the depth thereof is shallow, the decrease in the amount of light due to the absorption is minimized.

### [Carbon Dioxide]

In the present invention, it is preferable that the culturing be performed without intentionally using means for supplying carbon dioxide to the medium. That is, it is preferable that a method for supplying gas containing carbon dioxide to the medium through bubbling not be used. This is to prevent the biofilm (film-like structure or three-dimensional structure) formed of microalgae on the liquid surface from being destroyed due to bubbling. However, when the destruction is partially caused, carbon dioxide may be supplied through bubbling. In addition, the method for floating microalgae on a liquid surface through bubbling is disclosed in JP-A-2001-340847, JP-A-2007-160178, JP-A-62-213892, JP-A-1-131711, and the like. However, means for supplying carbon dioxide may also be employed in addition to forcibly floating microalgae on a liquid surface by the method. The method of not using the bubbling is extremely advantageous from the viewpoints that it is unnecessary to install gas piping for supplying carbon dioxide, it is generally difficult to secure land in the vicinity of a supply source of carbon dioxide, for example, a thermal power station or a steelworks, and it is extremely difficult to perform control for uniformly supplying carbon dioxide to a wide area. Moreover, the method of not using bubbling is extremely advantageous from the viewpoint that the method results in great reduction in cost since it is possible to directly use carbon dioxide in the gas phase.

In the present invention, it is preferable to use means for intentionally supplying carbon dioxide to the inside of a medium. However, a case, in which carbon dioxide is supplied to the inside of a medium such that carbon dioxide in the gas phase passes through microalgae on a liquid surface or a region in which microalgae do not exist, is defined as not corresponding to the means. There are many portions in which the liquid surface of a medium is directly brought into contact with gas containing carbon dioxide before a film-like structure formed of microalgae is formed on the liquid surface. In this case, carbon dioxide dissolves in the medium through the liquid surface. However, it is not considered that carbon dioxide is intentionally supplied in this case. In addition, carbon dioxide dissolves in the medium through a film-like structure or a three-dimensional structure even after the film-like structure or the three-dimensional structure is formed on the liquid surface. However, it is not considered that carbon dioxide is intentionally supplied to the medium in this case as well.

In the present invention, it is desirable to use carbon dioxide in the air from the viewpoint that it is advantageous in cost. However, it is also possible to use carbon dioxide having a higher concentration than that in the air. In this case, it is desirable to perform culturing using a closed culture container in order to prevent the loss of carbon dioxide due to diffusion. The concentration of carbon dioxide in the gas phase in this case is not particularly limited as long as it is possible to achieve the effect of the present invention, but is preferably greater than or equal to the concentration of carbon dioxide in the air and less than 20 volume%, more preferably 0.1 volume% to 15 volume%, and particularly preferably 0.1 volume% to 10 volume%.

The concentration of carbon dioxide in the air is generally understood to be about 0.04 volume%.

That is, the present invention is advantageous since it is possible to use carbon dioxide in the air. This is because it is easy to take up carbon dioxide in the gas phase since microalgae for which a large amount of growth is desirable are on the liquid surface. In this case, piping for carbon dioxide or bubbling of carbon dioxide is unnecessary, and therefore, it is possible to reduce the cost of producing algal bodies.

In addition, in the method of the present invention, the culturing is performed on the gas-liquid interface, and thus less death of algal bodies due to drying is exhibited.

### [Other Culturing Conditions]

The pH of a liquid medium (hereinafter, the liquid medium is also referred to as culture solution) immediately after starting the culturing is preferably within a range of 2 to 11, more preferably within a range of 5 to 9, and particularly preferably within a range of 5 to 7. This is because it is possible to favorably increase the proliferation rate of microalgae. In addition, in the medium such as a CSiFF03 medium, which is high in pH and easily generates deposits, a few deposits are generated at a pH on a weak alkali side, but hardly any deposits are generated at a weakly acidic pH. In addition, it is desirable to perform the culturing under weakly acidic conditions from the viewpoint that the number of algae increases on the liquid surface compared to algae on the bottom surface. In addition, it is desirable to select the pH depending on the kinds of microalgae from the viewpoint that a favorable pH changes depending on the kinds of microalgae. The pH of the liquid medium is a pH at the time of starting culturing. In addition, in some cases, the pH immediately after starting the culturing and the pH after starting the culturing changes in accordance with the proliferation of microalgae. Therefore, in the present invention, the pH of the liquid medium immediately after starting the culturing may be different from the pH at the time of collecting the microalgae.

The culture temperature can be selected in accordance with the kinds of microalgae, but is preferably 0°C to 90°C, more preferably 0°C to 50°C, still more preferably higher than or equal to 0°C and less than 40°C, still more preferably higher than or equal to 15°C and less than 40°C, and particularly preferably higher than or equal to 20°C and less than 40°C. When the culture temperature is higher than or equal to 20°C and less than 40°C, the proliferation rate of microalgae is sufficiently high, and the proliferation rate is particularly highest when the culturing is performed at 37°C.

The lower limit initial alga body concentration of microalgae is not particularly limited since the microalgae can proliferate as time passes as long as there are algal bodies in the culture solution, but is preferably higher than or equal to 1 piece/mL, more preferably higher than or equal to 1000 cells/mL, and still more preferably higher than or equal to 1 x 10⁴ cells/mL. The upper limit initial alga body concentration of microalgae is not particularly limited since the microalgae can proliferate at any high concentration, but is preferably lower than or equal to 10000 x 10⁴ piece/mL, more preferably lower than or equal to 1000 x 10⁴ piece/mL, and still more preferably lower than or equal to 500 x 10⁴ cells/mL from the viewpoint that when the concentration is more than a certain concentration, the ratio of the number of algal bodies after the proliferation to the number of input algal bodies decreases as the concentration of the algal bodies becomes higher.

In the present invention, it is possible to mix a medium, which has been used for culturing once, with a medium newly prepared. By doing this, it is possible to reduce the amount of water used while there is a case where the growth quantity of microalgae decreases. In addition, a method of adding a high-concentration medium can be considered as a method for suppressing the decrease of the growth quantity of microalgae, and it is possible to use this method in the present invention.

In the present invention, the pre-culturing period in the case of performing the liquid surface-floating culture is preferably 1 day to 300 days, more preferably 3 days to 100 days, and still more preferably 7 days to 50 days.

In regards to the period of the culturing (liquid surface-floating culture), it is possible to continue the culturing as long as the microalgae grow, but in general, the period thereof is preferably 1 day to 100 days, more preferably 7 days to 50 days, and still more preferably 10 days to 30 days.

In the present invention, it is possible to add a substance, which has a buffer action, to a medium for maintaining a constant pH in the medium. In general, it is known that microalgae release various substances outside the bacterial cell in accordance with survival or proliferation. Therefore, it can be considered that the pH in the medium may change depending on the released substances and the environment may change to an environment in which the culturing of microalgae is not preferably performed. It is preferable to add the substance having a buffer action in order to avoid such a phenomenon. Furthermore, microalgae proliferate using carbon dioxide as a carbon source. Therefore, it can also be considered that the pH of a medium decreases in accordance with dissolution of carbon dioxide into the medium and the environment changes to an environment in which the culturing of microalgae is not preferably performed. It is preferable to add the substance having a buffer action in order to avoid this phenomenon also. A well known substance can be used as the substance having a buffer action and there is no restriction on its use. However, 4-(2-hydroxyethyl)-1-piperazineethanesulfonic acid (HEPES) buffer solution, a sodium phosphate buffer solution, a potassium phosphate buffer solution, or the like may be preferably used. The concentrations or the kinds of buffer substances can be determined in accordance with the culture environments of the microalgae.

### [Culture Container]

As the shape of culture container (culture pond) that can be used in the present invention, a well-known culture container having any form can be used as long as it is possible to maintain the suspended solution of microalgae. For example, it is possible to use a culture container having a cylindrical shape, a rectangular shape, a spherical shape, a plate shape, a tubular shape, and an irregular shape such as plastic bag. In addition, as the culture container that can be used in the present invention, various well-known methods using types such as an open pond type, a raceway type, and a tube type (J. Biotechnol., 92, 113, 2011) can be used. Examples of the forms that can be used as the culture container include culture containers disclosed in Journal of Biotechnology 70 (1999) 313-321, Eng. Life Sci. 9, 165-177 (2009). Among these, use of the open pond type or the raceway type is preferable in view of cost.

As the culture container that can be used in the present invention, any of an open type and a closed type can be used. However, a closed type culture container is preferably used in order to prevent microorganisms other than target microorganisms, or debris from being mixed in, to reduce evaporation of a medium, to prevent biofilm on a liquid surface from being destroyed or moved due to wind, and to prevent carbon dioxide from diffusing outside the culture container when using carbon dioxide having a concentration greater than or equal to the concentration of carbon dioxide in the air.

### [Light Source and Amount of Light]

As light sources that can be used for the light irradiation, any light sources can be used. However, it is possible to use sunlight, LED light, a fluorescent lamp, an incandescent lamp, xenon lamp light, a halogen lamp, and the like. Among these, it is preferable to use sunlight as natural energy, an LED having a good luminous efficiency, and a fluorescent lamp that can be simply used.

The amount of light is preferably 100 lux to 1,000,000 lux and more preferably 300 lux to 500,000 lux. The most preferable amount of light is 1000 lux to 200,000 lux. A larger amount of light is preferable in view of improvement of proliferation rate of microalgae. If the amount of light is greater than or equal to 1000 lux, the growth rate of microalgae is sufficiently high. If the amount of light is less than or equal to 200,000 lux, generation of light damage can be suppressed and increase of proportion, at which the proliferation rate of microalgae is decreased or microalgae become die, can be sufficiently suppressed.

The light may be radiated through any method such as continuous irradiation, and repetition of irradiation at a constant time interval and non-irradiation, but it is preferable that light be turned on and off at a time interval of 12 hours since this is close to a natural state. From results of tests, for the liquid surface-floating culture, it is preferable that continuous irradiation be performed immediately after starting of the culturing and that irradiation be performed by turning light on and off at a time interval of 12 hours from a middle stage of the culturing to a later stage of the culturing.

The wavelength of light is not particularly limited, and any wavelength can be used as long as the wavelength is a wavelength at which photosynthesis can be performed. A preferable wavelength is a wavelength of sunlight or a wavelength similar to that of sunlight. However, an example in which the growth rate of photosynthetic organisms is improved by radiating a single wavelength has been reported, and in the present invention, it is preferable to use such an irradiation method. Meanwhile, from the viewpoint of cost, irradiation using light without controlling the wavelength is more financially advantageous compared to irradiation using light having a single wavelength. Therefore, use of sunlight is most advantageous in viewpoint of cost.

### [Biofilm Formed on Liquid Surface]

The present invention also relates to a biofilm formed on a liquid surface by the method for culturing microalgae of the present invention.

The biofilm is generally a film-like structure and refers to a state in which microalgae are connected to each other and form the film-like structure. In order to connect the microalgae to each other, for example, substances (for example, polysaccharides) such as a matrix such as cells are released from the microalgae, which are connected to each other through a chemical action of the substances. That is, the biofilm is in a state in which microalgae are connected to each other to the extent that they are not separated from each other with the movement of a weak water current. In general, in many cases, such a film-like structure is described as a biofilm.

It is preferable that the biofilm according to the present invention be a film-like structure or a steric three-dimensional structure, to be described later, which is formed of AVFF007 strains of microalgae.

The biofilm according to the present invention may be a uniform film-like structure of aggregates of microalgae without any gaps over the entire culture container, and may be a steric three-dimensional structure in which a portion of such a film-like structure is formed in a shape of a rising bubble. In addition, such a portion of the steric three-dimensional structure may be a complicated structure further formed in a shape of a rising bubble. The phenomenon that a portion of the film-like structure rises in the bubble shape is observed in accordance with the progress of proliferation of microalgae. It is inferred that the phenomenon of rising in the bubble shape is caused by oxygen discharged through fixing of CO₂ during the proliferation of the microalgae. In addition, when forming the three-dimensional structure, the structure may be a structure in which many microalgae exist in a region close to a light source.

Several steric three-dimensional structures formed in the shape of a rising bubble may be formed in a culture container and the sizes thereof may be different from each other.

The existing location of microalgae is separated from a liquid surface and is close to a light source as the three-dimensional structure develops. This reduces the supply of water from the liquid surface and it is difficult to diffuse heat using light irradiation. As a result, the water content ratio decreases since the microalgae exist at a location away from the liquid surface. The decrease in the water content ratio enables simplification of a dehydration process when performing the oil extraction process after the collecting process, and therefore, this is advantageous with respect to cost reduction in production of biomass using microalgae. In general, a treatment of reducing the water content ratio of microalgae is performed using a centrifugal separator during the collecting process. However, in the collection method using the culturing method of the present invention, it is possible to obtain a water content ratio lower than that of the water content ratio of microalgae obtained by a centrifugal separator.

In addition, in some cases, a wrinkle-like structure appears in the film-like structure in accordance with the progress of proliferation of microalgae. The film-like structure may have such an accompanying structure.

Furthermore, in some cases, the film-like structure or the steric three-dimensional structure forms a pleated structure or a curtain-like structure in the medium in accordance with the progress of proliferation of microalgae. The film-like structure and the steric three-dimensional structure may have such an accompanying structure.

As described above, the film-like structure or the steric three-dimensional structure may be accompanied by the wrinkle-like structure, the pleated structure, and the curtain-like structure, or the film-like structure may be made to have a steric three-dimensional structure formed with the bubble-like structure. Employing such structures is preferable in view of increasing the quantity of algal bodies per unit area.

It is preferable that the film-like structure have an area to the extent that a piece of the film-like structure existing on the liquid surface does not escape from a substrate through the liquid surface while performing collection using the substrate. It is more preferable that there be no gaps in the film-like structure over the entire culture container. For example, such an area can be larger than or equal to 1 cm² and preferably larger than or equal to 10 cm². The area is particularly preferably larger than or equal to 100 cm². The upper limit of such an area is not particularly limited as long as the area is smaller than or equal to the area of the liquid surface of the culture container.

The thickness of film-like structure is generally within a range of 1 µm to 10000 µm, preferably within a range of 1 µm to 1000 µm, and more preferably 10 µm to 1000 µm.

In a case in which the biofilm according to the present invention is a steric three-dimensional structure formed in a shape of a rising bubble in a portion or a plurality of portions of film-like structure, the height of the steric three-dimensional structure with the liquid surface of the medium as a reference is generally within a range of 0.01 mm to 100 mm, preferably within a range of 0.1 mm to 20 mm, and more preferably within a range of 5 mm to 20 mm.

The weight of dry alga bodies of the biofilm according to the present invention per unit area is preferably greater than or equal to 0.001 mg/cm², more preferably greater than or equal to 0.1 mg/cm², and particularly preferably greater than or equal to 1 mg/cm². The most preferable weight of the dry alga bodies is greater than or equal to 5 mg/cm². This is because it is expected that if the weight of the dry alga bodies per unit area is great, the collected amount of biomass such as oil will be greater. In general, the weight of the dry alga bodies of the biofilm per unit area is less than or equal to 100 mg/cm².

In addition, the film density of the microalgae in the biofilm according to the present invention per unit area is preferably greater than or equal to 100,000 pieces/cm2, more preferably greater than or equal to 1,000,000 pieces/cm², and particularly preferably greater than or equal to 10,000,000 pieces/cm². When the film density of the microalgae in the biofilm per unit area is less than 100,000 pieces/cm², the formation of the biofilm on the liquid surface cannot be confirmed and the recovery property of microalgae deteriorates. In contrast, when the film density of the microalgae in the biofilm per unit area is greater than or equal to 10,000,000 pieces/cm², due to the high film density, a strong biofilm is formed on the liquid surface and the recovery property improves. The greater the film density, the more preferable, and therefore, the upper limit of the film density of the microalgae in the biofilm per unit area is not particularly limited. In general, the film density is less than or equal to 10 trillion pieces/cm².

It is preferable that the biofilm according to the present invention have a low water content ratio in view of saving labor in a process of removing water and in view of cost reduction. Specifically, the water content ratio of the biofilm after the collection is preferably less than or equal to 95 mass%, more preferably less than or equal to 90 mass%, still more preferably less than or equal to 80 mass%, and particularly preferably less than or equal to 70 mass%. In general, the water content ratio of the biofilm is greater than or equal to 50 mass%. The water content ratio is obtained by dividing a value, which is obtained by subtracting the weight of algal bodies after drying from the weight of algal bodies before drying, by the weight of algal bodies before the drying, and then by multiplying by 100.

In addition, it is preferable that the biofilm according to the present invention have a high oil content in view of usefulness as biomass. Specifically, the oil content of the biofilm in the dry alga bodies is preferably higher than or equal to 5 mass%, more preferably higher than or equal to 10 mass%, and higher than or equal to 10 mass%, and particularly preferably higher than or equal to 15 mass%. In general, the oil content of the biofilm per dry alga body is lower than or equal to 80 mass%.

Microalgae capable of forming a biofilm, which has the structure, the area in the above-described range, the thickness, the weight of dry alga bodies per unit area, the water content ratio, and the oil content which are described above, on a liquid surface are preferable as the microalgae of the present invention for the above-described reasons.

### (Collection of Biofilm Formed on Liquid Surface)

In the present invention, a biofilm formed on a liquid surface (a film-like structure formed of microalgae or a steric three-dimensional structure formed in a shape of a rising bubble in a portion or a plurality of portions of the film-like structure) can be easily collected using a substrate.

Specifically, in the biofilm formed on the liquid surface in the present invention as described above, microalgae are connected to each other through a chemical action through substances such as a matrix between cells, and the microalgae are connected to each other to the extent that they are not separated from each other with the movement of a weak water current. Therefore, it is possible to easily collect the biofilm in a manner of being deposited on the substrate.

The collection of the biofilm from the liquid surface-floating culture, which is described above, will be described with reference to the accompanying drawings.

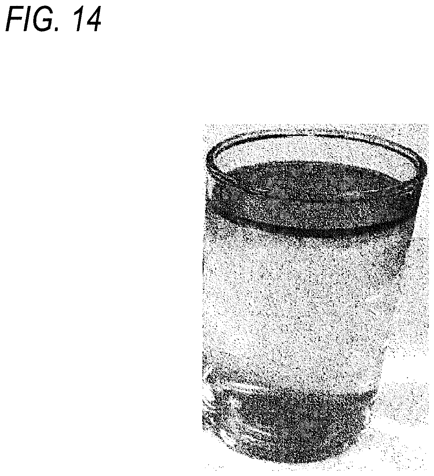

Fig. 14 is a view showing a photograph of liquid surface-floating culture. As shown in Fig. 14, the microalgae of the present invention are characterized by a biofilm (film-like structure in Fig. 14) formed of microalgae being formed on a liquid surface.

As described above, Fig. 12 is a state in which a film-like structure formed of microalgae floating on a liquid surface is collected using a glass substrate (slide glass (76 mm x 26 mm)) after forming the biofilm (film-like structure in the example of the drawing) on the liquid surface. The biofilm is collected by obliquely inserting the long side of the glass substrate into the biofilm on the liquid surface and advancing the glass substrate in a left direction as it is, and by depositing the algae on the liquid surface on the glass substrate. The glass substrate is moved to the left side from the right side in Fig. 12. However, the movement direction of the substrate may be reversed (that is, the movement of the glass substrate from the left side to the right side) or the collection may be performed several times. When performing the collection several times, the substrate may be used in a state in which the biofilm is adhered thereto; a substrate after removing the biofilm on the substrate from the top of the substrate may be used; or a new substrate may be used. In addition, only one sheet of the substrate is drawn in Fig. 12, but plural sheets of the substrate may be used at the same time. In addition, it is possible to freely set the angle, the size and the speed of the substrate depending on the purpose.

In regards to the timing of collecting the biofilm on the liquid surface, it is possible to collect the biofilm in a state in which the liquid surface in the culture container is partially covered with the biofilm. However, it is preferable to collect the biofilm when the liquid surface in the culture container is completely covered with the biofilm in view of obtaining a large number of algal bodies of the microalgae. In addition, the collection may be performed after continuing culture for a while after the liquid surface is completely covered with the biofilm.

When collecting the biofilm using the substrate, it is preferable that the substrate have the same length as that of a side of the liquid surface in the culture container. This is because if the substrate has a shorter length than that of the side, the recovery amount decreases, the microalgae on the liquid surface deviate from the subject to be collected by passing through the space between the substrate and the wall of the culture container, and the collected microalgae on the substrate move to an opposite side from the progressing direction of the substrate. In addition, it is preferable that the substrate have the same length as that of a short side of the culture container.

Fig. 13 shows a state of a collected biofilm deposited so as to be folded over the glass substrate.

The substrate may have any shape such as a film shape, a plate shape, a fibrous form, a porous form, a convex shape, and a wavy shape. However, it is preferable that the substrate have a film shape or a plate shape in view of easy deposition of the biofilm or the like and easy collection of the microalgae from the substrate.

In addition, it is possible to use other substrates such as nylon film as the substrate in addition to the slide glass as the glass substrate. In addition, the size of the substrate can be appropriately changed depending on the size of the culture container.

Any well-known method can be used for collecting the biofilm from the substrate as long as the method can separate the biofilm from the substrate. For example, a method using gravity, a method of peeling off the biofilm from the substrate using a cell scraper or the like, a method of using a stream of water, and a method of using ultrasonic waves can be used. Among these, the method of using free fall due to gravity or the method of using the cell scraper or the like is preferable. This is because these methods are efficient since biofilm is not diluted by a solvent or a liquid medium. In addition, it is also possible to additionally collect the algae remaining on the substrate using the cell scraper or the like after collecting the biofilm using free fall due to gravity.

### [Collecting process]

The collecting process is a process of peeling off a biofilm from a first substrate after the biofilm (film-like structure or three-dimensional structure) formed of microalgae on a liquid surface is transferred to a first substrate as shown in (d) to (e) of Fig. 11, and a process of collecting the biofilm (film-like structure or three-dimensional structure) formed of the microalgae on the liquid surface using a second substrate as shown in (f) to (g) of Fig. 11.

The present disclosure also relates to a method for collecting biofilm in which the biofilm formed on a liquid surface is collected by transferring the biofilm to a substrate (first substrate).

In addition, the present disclosure also relates to a method for collecting biofilm in which the biofilm formed on a liquid surface is collected by depositing the biofilm on a substrate (second substrate).

Any well-known method can be used for collecting the biofilm from the first substrate as long as the method can separate the biofilm from the first substrate. For example, a method of peeling off the biofilm from the substrate using a cell scraper or the like, a method of using a stream of water, and a method of using ultrasonic waves can be used. Among these, the method of using the cell scraper or the like is preferable. This is because other methods are inefficient since the biofilm is diluted by a medium or the like and it is necessary to re-concentrate the biofilm.

Any well-known method can be used for collecting the biofilm from the second substrate as long as the method can separate the biofilm from the second substrate. For example, a method using gravity, a method of peeling off the biofilm from the substrate using a cell scraper or the like, a method of using a stream of water, and a method of using ultrasonic waves can be used. Among these, the method of using free fall due to gravity or the method of using the cell scraper or the like is preferable. This is because other methods are inefficient since the biofilm is diluted by a medium or the like and it is necessary to re-concentrate the biofilm. In addition, it is possible to collect algae remaining on the second substrate using the cell scraper or the like after collecting the biofilm using free fall due to gravity.

### [Collection of Entire Quantity]

Collection of the entire quantity is collecting all microalgae in a culture container. That is, the entire biofilm (film-like structure or three-dimensional structure) formed of deposited microalgae on a liquid surface is collected. Such a method can be performed when finishing the culturing, when culturing other microalgae, when changing a medium in a culture container, and the like. After performing transfer of a biofilm formed of microalgae on a liquid surface using a first substrate or collection using a second substrate, the medium may be removed and the remaining microalgae on the bottom surface of the culture container may be collected. Furthermore, all of the microalgae in the culture container may be collected through a well-known method. Examples of the well-known method include collection using a filter and collection using a coagulant.

For example, it is possible to collect the microalgae on the bottom surface of the culture container after collecting the biofilm of the microalgae on the liquid surface.

In addition, after collecting the biofilm formed of the microalgae on the liquid surface, the culture solution in the culture container may be removed and the microalgae on the bottom surface of the culture container or on the surface of the first substrate may be collected.

### [Transfer of Biofilm]

The transfer referred to herein is a kind of adhesion and is substantially adhesion without proliferation. In the present invention, the transfer refers to an operation of substantially collecting the biofilm formed on the liquid surface by transferring it onto the surface of the first substrate as it is using the first substrate.

As shown in (d) of Fig. 11, the transfer of the biofilm is a process of transferring the biofilm formed on the liquid surface to the surface of the first substrate using the first substrate. In the drawing, the collection process is performed in a state in which the biofilm is formed on the entire surface in the culture container. In the present invention, the collection process may be performed in such a state or also in a state in which the biofilm formed of microalgae partially exists. In addition, when performing the culturing by the method of the present invention, in some cases, the biofilm formed on the liquid surface forms a wrinkle-like shape or is folded and piled, or a pleated film-like structure formed of microalgae grows like an aurora (curtain shape) in the liquid. In the present invention, it is possible to perform the collection even in such a state, and a collecting method and a culturing method using such a method can be included in the present invention.

### [Substrate]

The substrate referred to herein is a substrate which is used in (d) of Fig. 11 or (f) of Fig. 11 and is used for transferring or collecting a biofilm formed of microalgae on a liquid surface.

The surface of a first substrate and the surface of a second substrate refer to every surface of the substrate including the upper surface of the substrate, the bottom surface of the substrate, and the side surface of the substrate. In a case in which the microalgae cannot come out of the culture solution from a layer formed between the substrate and the surface of the culture container or the like due to contact with the culture container or the like even if the microalgae are adhered to these surfaces, this is not referred to as the surface of the present invention.

The shape of the substrate may have a film shape, a plate shape, a fibrous form, a porous form, a convex shape, and a wavy shape. However, it is preferable that the substrate have a film shape or a plate shape in view of easy adhesion, deposition, transfer, or the like and easy collection of the microalgae from the substrate.

The first substrate and the second substrate may have the same shape as each other or have a different shape from each other. It is preferable that the area of the first substrate and the second substrate be smaller than that of the liquid surface of the culture solution of the culture container.

### [Material]

The materials of the culture container, the first substrate, and the second substrate that can be used in the present invention are not particularly limited, and well-known materials can be used. For example, it is possible to use a material formed of an organic polymer compound, an inorganic compound, or a composite thereof. In addition, it is also possible to use a mixture thereof.

Polyethylene derivatives, polyvinyl chloride derivatives, polyester derivatives, polyamide derivatives, polystyrene derivatives, polypropylene derivatives, polyacrylic derivatives, polyethylene terephthalate derivatives, polybutylene terephthalate derivatives, nylon derivatives, polyethylene naphthalate derivatives, polycarbonate derivatives, polyvinylidene chloride derivatives, polyacrylonitrile derivatives, polyvinyl alcohol derivatives, polyethersulfone derivatives, polyarylate derivatives, allyl diglycol carbonate derivatives, ethylene-vinyl acetate copolymer derivatives, fluorine resin derivatives, polylactic acid derivatives, acrylic resin derivatives, ethylene-vinyl alcohol copolymers, ethylene-methacrylic acid copolymers, and the like can be used as the organic polymer compound.

Glass, ceramics, concrete, and the like can be used as the inorganic compound.

Alloys such as iron, aluminum, copper, or stainless steel can be used as a metallic compound.

Among these, it is preferable that a portion of the materials of the first substrate, the second substrate, or the culture container be formed of at least one selected from glass, polyethylene, polypropylene, nylon, polystyrene, vinyl chloride, and polyester.

In addition, the materials of the culture container, the first substrate, and the second substrate may be the same as each other or different from each other.

In addition, when using a closed type culture container, the light receiving surface may be made of a material through which light is transmitted, and a transparent material is more preferable.

### [Surface Unevenness of Substrate]

In the present invention, it is possible to form unevenness on the substrate. In some cases, the surface area of the adhesion increases and the adhesion, the transfer, and the collection stability of algal bodies improves by forming the unevenness on the surface of the substrate.

### [Bottom Surface]

The bottom surface referred to in the present invention can include portions other than the bottom surface of the culture container. Examples thereof can include the side surface of the culture container or the surface of an immersed sensor or the like in the middle of the liquid. The effect of the side surface becomes greater as the culture container becomes smaller.

### [Biomass and Oil]

The present invention also relates to biomass and methods to collect oil obtained from the biofilm of the present invention which is formed of microalgae on a liquid surface.

Herein, "biomass" refers to a renewable organic resource derived from organisms excluding a fossil resource, and examples thereof include materials, food products, materials, fuel, and resources derived from organisms.

Herein, "oil" refers to a flammable fluid substance, is a compound mainly formed of carbon and hydrogen, and is a substance occasionally containing oxygen, nitrogen, and the like. In general, the oil is a mixed material and is a substance which is extracted using a low-polarity solvent such as hexane or acetone. The composition thereof is formed of hydrocarbon compounds, fatty acids, triglycerides, or the like. In addition, the composition thereof is esterified to be used as biodiesel.

The method for collecting the biomass and the oil contained in the biofilm according to the present invention is not particularly limited as long as the method does not impair the effect of the present invention.

As a general collecting method of the oil as an example of the biomass, after obtaining dry alga bodies by heat-drying the biofilm, cell-disruption is performed as necessary to extract the oil using an organic solvent. In general, the extracted oil contains impurities such as chlorophyll, and therefore, it is necessary to perform purification. There is a case of performing purification through silica gel column chromatography or performing purification through distillation (for example, a distillation method disclosed in JP-T-2010-539300).

In addition, there is a method of extracting the oil in the algal bodies using an organic solvent after crushing the microalgae through an ultrasonic treatment or crushing the microalgae using protease or an enzyme after a solution of high-concentration microalgae is prepared (for example, a method disclosed in JP-T-2010-530741).

In this manner, the biofilm according to the present invention is useful as biomass fuel. That is, the present invention also relates to a method for producing biomass fuel in which the biofilm collected by the method for collecting the biofilm according to the present invention is used as fuel.

### [Dry Alga Bodies]

The dry alga bodies in the present invention are obtained by drying biofilm according to the present invention.

The method of drying the biofilm is not particularly limited as long as the method can remove water from the biofilm. Examples thereof include a method of sun-drying the biofilm; a method of heat-drying the biofilm; a method of freeze-drying the biofilm; and a method of blowing dry air onto the biofilm. Among these, the freeze-drying drying method is preferable in view of being capable of suppressing decomposition of components contained in the biofilm and the heat-drying method is preferable in view of being capable of efficiently perform the drying in a short period of time.

### Examples

The present invention will be further described in detail with reference to the following examples, but the present invention is not limited to the following examples.

### [Example 1]

### <Formation of Biofilm (Film-like Structure) on Liquid Surface using AVFF007 Strains>

The total quantity of a solution containing AVFF007 strains for which microorganism purification was performed was put into a tube for homogenizing with a 5 mL capacity (TM-655S, Tomy Seiko Co., Ltd.), the tube was set in a beads cell disrupter (MS-100, Tomy Seiko Co., Ltd.), and then, a homogenization treatment lasting for 20 seconds was performed 3 times at 4200 rpm to obtain a suspended solution of the AVFF007 strains.

The total quantity of the solution was put into a conical flask into which 40 mL of a CSi medium was put and stationary culture (hereinafter, also referred to as pre-culture) was performed in a desiccator under a 5% CO₂ atmosphere. The culturing was performed at a temperature of 23°C and an amount of light of 2000 lux.

The solution containing the microalgae was collected from the conical flask and was put into a tube for homogenizing with a 5 mL capacity, the tube was set in a beads cell disrupter, and then, a homogenization treatment lasting for 20 seconds was performed 3 times at 4200 rpm. Here, beads for the cell disrupter were not used.

50 µL of a suspended liquid of microalgae was collected from the solution and was put into a microtube for homogenizing to which 950 µL of a CSi medium was added in advance, the microtube was set in a beads cell disrupter, and then, a homogenization treatment was performed for 20 seconds at 5500 rpm. Here, beads for the cell disrupter were not used. About 10 µL of the solution was collected and the concentration of algal bodies was counted on a hemocytometer. The result was that the concentration of the algal bodies in the tube for homogenizing with a 5 mL capacity was 695 x 10⁴ cells/mL.

50 mL of CSi medium was put into a centrifugal settling tube with a 50 mL capacity (MS-57500, Sumitomo Bakelite Co., Ltd.), 720 µL of the suspended liquid of microalgae was added to the tube with a 5 mL capacity, the mixture was well stirred, and then, the stirred mixture was added to a 6 hole plate at 8 mL per hole.

The culturing was performed using a plant bioshelf for tissue culture (AV152261-12-2, Ikeda Scientific Co., Ltd.) and by placing the 6 hole plate in a vacuum desiccator (1-070-01, As One Corporation). Stationary culture was performed at 4000 lux and a temperature of 23°C under the condition in which turning on and off of the light irradiation was performed once every 12 hours (hereinafter, also referred to as "12 hours on-off irradiation" or 12 hours on-off control"). In addition, the CO₂ concentration in the vacuum desiccator was 10%.

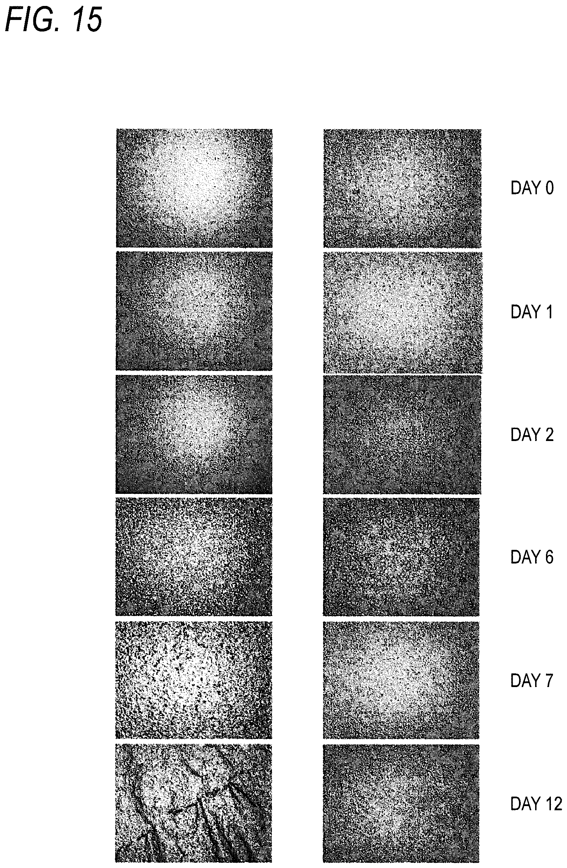

The 6 hole plate was taken out of the vacuum desiccator during the liquid surface-floating culture to directly observe the top of the liquid surface using an optical microscope. The observation result is shown in Fig. 15. The left side of Fig. 15 is a result of observation using the optical microscope at magnification of 4 and the right side of Fig. 15 is a result of observation using the optical microscope at magnification of 40. Until day 1 of the liquid surface-floating culture, the AVFF007 strains mostly did not exist on the liquid surface, a few AVFF007 strains started to appear on day 2 of the culturing, and the top of the liquid surface was mostly covered by the AVFF007 strains as long as it was observed by a microscope on day 6 of the culturing. It was possible to visually and clearly check the formation of a film-like structure on the liquid surface on day 7 of the culturing. The AVFF007 strains on the liquid surface proliferated to be a state of being wrinkled resulting in increase of the surface area for proliferation on day 12 of the culturing.

As is obvious from the above results, the AVFF007 strains as microalgae of the present invention can form the biofilm on the liquid surface.

### [Example 2]

### <Formation of Biofilm (Steric Three-dimensional Structure Formed in Shape in Rising Bubble in Portion or Plurality of Portions of Film-like Structure) on Liquid Surface using AVFF007 Strains>

Pre-culture of AVFF007 strains of microalgae was performed for 28 days using the same method as that of Example 1. After the pre-culture, the AVFF007 strains on the liquid surface were collected and the total quantity of the AVFF007 strains was put into a tube for homogenizing with a 5 mL capacity to prepare 3 mL of a suspended solution of the AVFF007 strains. The concentration of the algal bodies of the prepared suspended solution of the AVFF007 strains was counted similarly to Example 1, and 32389 x 10⁴ cells/mL was the result.

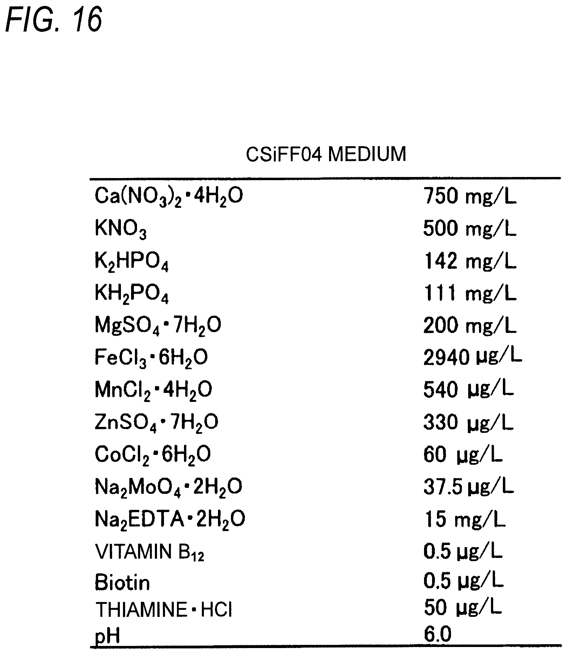

350 mL of a CSiFF04 medium shown in Fig. 16 and 540 µL of the suspended solution of the AVFF007 strains of the algae were added to a conical glass flask with a 500 mL capacity to prepare a solution with a concentration of the AVFF007 strains of 50 x 10⁴ cells/mL.

The solution was put into containers which was a 28-type case made of polystyrene (of which the material was polystyrene and manufactured by As One Corporation) at 40 mL (1.5 cm water depth) for each container to prepare 3 containers thereof. All the 28-type cases made of polystyrene were put into a vacuum desiccator without being covered with lids and the door of the vacuum desiccator was closed while setting a 5% CO₂ atmosphere. Carbon dioxide was newly prepared every evaluation day.

The vacuum desiccator was placed in a plant bioshelf to perform the liquid surface-floating culture by the stationary culture at 15000 lux and 23°C under the conditions in which turning on and off of the light irradiation was performed once every 12 hours.

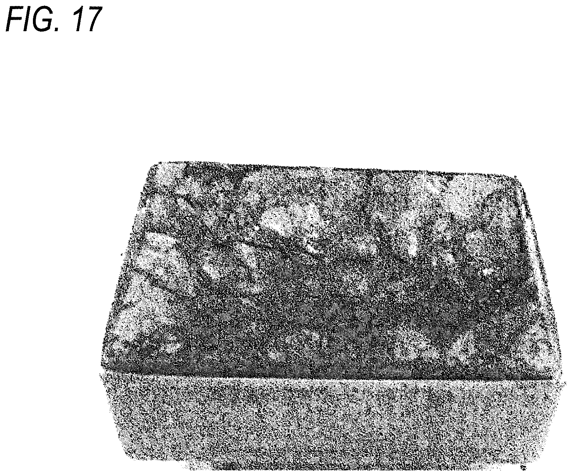

After performing the liquid surface-floating culture for 4 days, 7 days, and 10 days, the dry weight was measured by collecting the biofilm on the liquid surface and heat-drying the biofilm using the same method as that described in Fig. 12. As shown in Fig. 17, the biofilm on the liquid surface after performing the liquid surface-floating culture for 14 days formed three-dimensional structures in which steric structures, which were formed in a shape of a rising bubble, overlapped each other. In addition, the water content ratio of the microalgae positioned away from the liquid surface was considered to be reduced (dried), and the color of the microalgae had changed from a yellow color to a light brown color. It is known that the color of oil-accumulating algae such as Botryococcus changes from a green color to a brown color or a yellow color, a red color, and the like if oil accumulates. Accordingly, it was considered that the AVFF007 strains in the above-described biofilm deposited the oil.

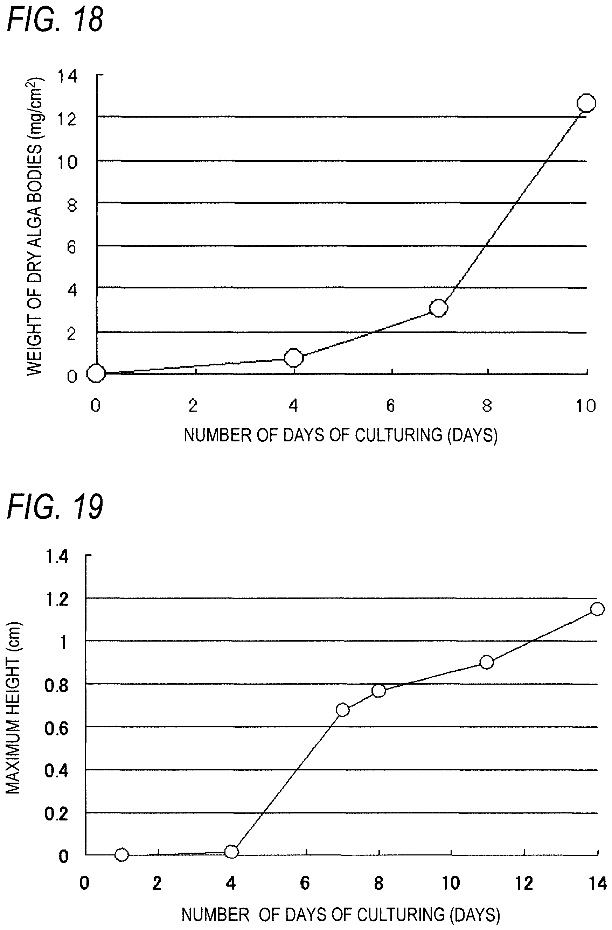

The relationship between the number of days of the liquid surface-floating culture and the quantity of dry alga bodies of the biofilm for each number of days of the liquid surface-floating culture is shown in Fig. 18. The weight of the dry alga bodies reached 12.6 mg/cm² 10 days after the start of the liquid surface-floating culture. The proliferation rate of the dry alga bodies reached 13 g/m²/day for 10 days, 20 g/m²/day for periods between day 4 to day 10, and 32 g/m²/day for periods between day 7 to day 10. As is obvious from Fig. 18, the logarithmic growth phase of the AVFF007 strains on the liquid surface in Example 2 was the periods between day 4 to day 10.

In addition, the relationship between the number of days of the liquid surface-floating culture and the maximum height (cm) of the biofilm for each number of days of liquid surface-floating culture is shown in Fig. 19. The maximum height of the biofilm is a value obtained by measuring the height of the biofilm at a position at which the height of the biofilm is at a maximum from the liquid surface in the biofilm formed on the liquid surface. As is obvious from Fig. 19, the maximum height steeply increased after day 4, and therefore, it can be seen that the structure of the biofilm changed from the film-like structure to the three-dimensional structure in this period.

Furthermore, the water content ratio of the microalgae existing at a position away from the liquid surface in the formed biofilm seemed to be reduced (dried). Therefore, it was considered that the water content ratio of the biofilm in Example 2 was reduced and the water content ratio thereof was calculated. The water content ratio of the biofilm was obtained by dividing a value, which was obtained by subtracting the weight of algal bodies after drying from the weight of algal bodies before drying, by the weight of algal bodies before the drying, and then by multiplying by 100.

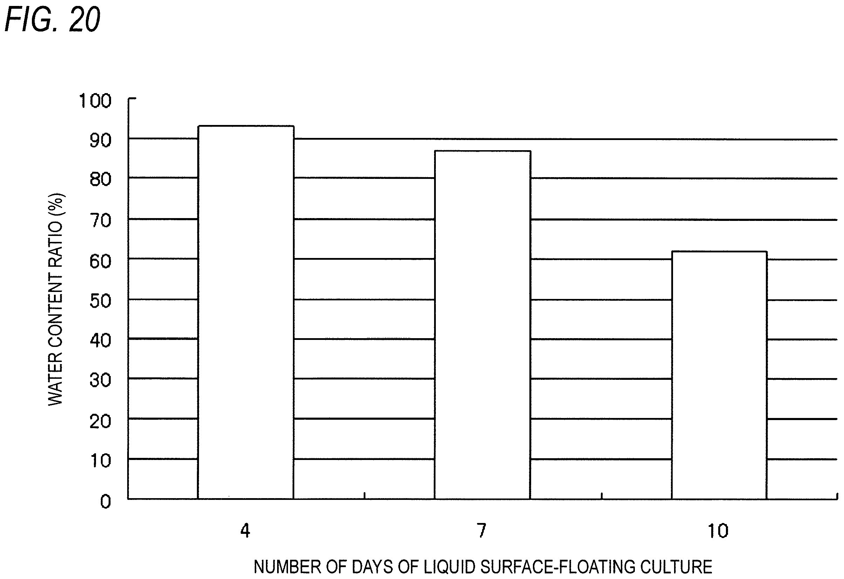

The relationship between the number of days of the liquid surface-floating culture and the water content ratio of the biofilm for each number of days of liquid surface-floating culture is shown in Fig. 20. In general, the water content ratio of algal bodies which can be collected by a centrifugal separator is about 90 mass% after collecting the microalgae deposited on the bottom surface of the culture container using a coagulant after performing the culturing by the floating culture. In the case of the AVFF007 strains of the microalgae, the water content ratio becomes about 90 mass% only by performing the collection using the substrates. Furthermore, as the liquid surface-floating culture progresses, the water content ratio of the biofilm collected using the substrates after the three-dimensional structure developed on the liquid surface and the water in the microalgae positioned away from the liquid surface started to evaporate (being dried) was further reduced.

In this manner, it is possible to reduce the water content ratio of the biofilm, which is collected by the liquid surface-floating culture method in the present invention, further than that of the collected substance of the microalgae which was obtained using the centrifugal separator (in general, about 90 mass% of the water content ratio). In other words, the biofilm formed of the microalgae of the present invention on the liquid surface is easily collected and the water content ratio thereof is reduced, and thus, it is possible to save labor in the process of removing water. Accordingly, it is possible to expect reduction in cost in a process such as a continuous oil extraction process (including the drying process).

### [Example 3]

### <Content of Oil When Performing Liquid Surface Culture]

A solution containing AVFF007 strains for which microorganism purification was performed was put into a conical flask with a 100 mL capacity into which 40 mL of a mixed medium (volume ratio of 1:1) of a C medium and a CSi medium was put and which was sterilized by dry heat, the flask was placed in a plant bioshelf for tissue culture, and stationary culture (hereinafter, also referred to as pre-culture) was performed at 23°C under continuous light irradiation at 4000 lux.

The solution containing the microalgae was collected from the conical flask and was put into a tube for homogenizing with a 5 mL capacity, the tube was set in a beads cell disrupter, and then, a homogenization treatment lasting for 20 seconds was performed 3 times at 4200 rpm without using beads for the cell disrupter.

50 µL of a suspended liquid of the microalgae was collected from the solution and was put into a microtube for homogenizing to which 950 µL of a CSi medium was added in advance, the tube was set in the beads cell disrupter, and then, a homogenization treatment lasting for 20 seconds was performed at 5500 rpm without using beads for the cell disrupter. About 10 µL of the solution was collected and the concentration of algal bodies was counted on a hemocytometer. The result was that the concentration of the algal bodies in the tube for homogenizing with a 5 mL capacity was 4975 x 10⁴ cells/mL.

50 mL of CSi medium was put into a centrifugal settling tube with a 50 mL capacity, 361.8 µL of the suspended liquid of microalgae was added to the tube with a 5 mL capacity, the mixture was well stirred, and then, the stirred mixture was added to a 6 hole plate at 8 mL per hole. The culturing was performed on a plant bioshelf for tissue culture under continuous light irradiation using a fluorescent lamp at 4000 lux. Room temperature was set to 23°C and the culturing performed was stationary culture.

After 30 days, the culturing was stopped and the biofilm on the liquid surface was collected using a nylon film.

50 mL the biofilm obtained by the above-described method was put into a centrifugal settling tube with a 50 mL capacity, the tube was set in a centrifugal spin dryer (VC-96R, Taitec Corporation), and a centrifugal treatment was performed at 2000 r/min (440 x g). The total quantity of the obtained deposits was put into a 2 mL sample bottle, which was made of glass and the weight of which was measured in advance, and the centrifugal treatment was performed again using the centrifuge. Then, the bottle was dried in a drying oven which was a ventilation constant-temperature oven (DKM600, Yamato Scientific Co., Ltd.) of which the temperature was set to 100 °C after removing a supernatant.

After the drying, the sample bottle containing the dried microalgae was weighed and the dry weight of the microalgae was obtained by subtracting the weight of the empty bottle and the weight of solid components in the medium which was diluted by 1/10 calculated from the amount of solvent. The amount of solvent was estimated from the difference between the weight before performing the heat drying and weight after performing the heat drying.

1 mL of a 5% hydrochloric acid-methanol mixed solution was added to the above-described sample bottle, and the mixture was reacted using a ventilation constant-temperature oven which was set to 100°C.

After being cooled to room temperature, the total amount of the mixture was transferred to a sample bottle with a 10 mL capacity, and 1.5 mL of hexane and 0.5 mL of distilled water were further added thereto. After being strongly stirred using a vortex mixer, the mixture was separated into two phases by allowing the mixture to stand, and a hexane phase was put into a sample bottle with a 2 mL capacity which was weighed in advance.

The above-described sample bottle with a 2 mL capacity was set in a centrifugal spin dryer, and organic solvent was evaporated under reduced pressure while centrifuging the mixture at 2000 r/min (440 x g).

After the evaporation, the mixture was heated in a ventilation constant-temperature oven which was set to 55°C, and then cooled to room temperature. Then, the weight of the sample bottle with a 2 mL capacity was measured and the difference between the weight thereof and the weight when the sample was not put therein was set as the weight of oil. In addition, the oil content in the dry alga bodies was calculated by dividing the weight of oil by the weight of the dry alga bodies.

The oil content was 11.72 mass% in the case of the AVFF007 strains.

### [Example 4]

### <Analysis Results of Oil Component>

A C medium, which was prepared so as to have 1 x 10⁴ cells/mL of the AVFF007 strains subjected to microorganism purification, was put into a 5 L water tank having a width of 60 cm, a depth of 45 cm, and a height of 45 cm, and liquid surface-floating culture through stationary culture was performed under the conditions of a temperature of 25°C and a light irradiation intensity of 65 µmol/m²/s.

After 30 days from the start of the culturing, the biofilm on the liquid surface was collected by being adhered to Parafilm (that is, the culturing method using the first substrate), and the collected substance was subjected to centrifugal operation for 10 minutes at 6000 x g and another 10 minutes at 8000 x g. Then, a supernatant was removed and freeze-drying was subsequently performed.

The obtained dried substance was crushed using a mortar and pestle and 4 mL of hexane was added thereto. Then, the total amount of the mixture was transferred to a centrifugal settling tube and was subjected to centrifugal operation for 10 minutes at 1500 x g. A hexane phase of the supernatant was collected and was put into a separate container. A 5% hydrochloriic acid -methanol mixed solution was added to the solid body and a heat treatment was performed for 1 hour at 100°C. Then, the temperature thereof was lowered to room temperature and hexane and distilled water were added thereto. After stirring the solution, centrifugal operation was performed for 5 minutes at 8500 x g and a water phase was removed. Then, distilled water was added to the remaining hexane phase again and the mixture was stirred. Centrifugal operation was subsequently performed for 5 minutes at 8500 x g. The hexane phase of the supernatant was mixed with the hexane phase which was collected before, and analysis by GC-MS was performed.

The conditions of the GC-MS analysis were such that the temperature was maintained at 90°C for 1 minute, a Rtx-5MS column (Restek Corporation) was used, and the temperature was increased at a rate of 10°C/min.

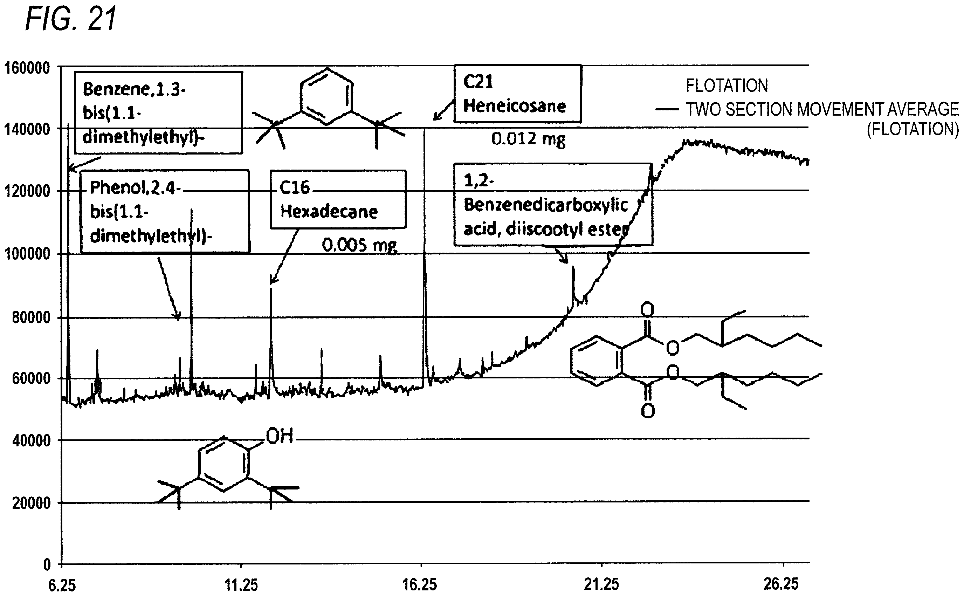

The results are shown in Fig. 21. It was obvious that the resultant mainly contains C16 and C21 oil components.

In addition, the used oil content which was calculated from the quantity of the dry alga bodies after removing the obtained solvent of the hexane phase was 25 mass%.

### [Example 5]

### <Collection of Biofilm Formed on Liquid Surface>

A solution at an alga body concentration of 50 x 10⁴ cells/mL was prepared by dispersing AVFF007 strains in 220 mL of a CSiFF04 medium using the same method as that in Example 1 except for using the CSiFF04 medium as a medium. The total quantity of the prepared solution was put into an 8-type styrene rectangular case.

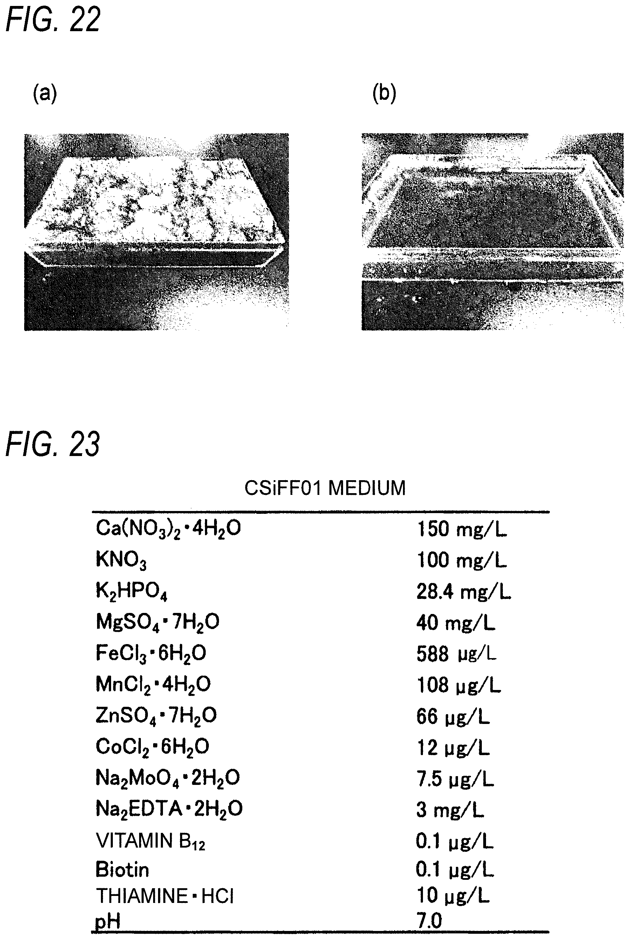

The case was put into a vacuum desiccator without being covered with a lid, the desiccator was placed in a plant bioshelf for tissue culture, and stationary liquid surface-floating culture was performed at 23°C and 15000 lux (12 hours on-off irradiation) using a fluorescent lamp under a 5% CO₂ atmosphere. After performing the culturing for 21 days, the 8-type styrene rectangular case was taken out of the vacuum desiccator. The state of the biofilm formed on the liquid surface is shown in (a) of Fig. 22. As shown in (a) of Fig. 22, the microalgae proliferated on the liquid surface in a rising manner to form a steric three-dimensional structure.

Next, a nylon 6 film having the same length as that of a short side of the case was used to collect the biofilm (steric three-dimensional structure) on the liquid surface using the method shown in Fig. 12. The state of the liquid surface after the collection is shown in (b) of Fig. 22. As shown in (b) of Fig. 22, no microalgae on the liquid surface were visually recognized at all. Furthermore, the collection of the microalgae on the liquid surface by the transfer was attempted using a polyethylene film, but the collected amount was 0 g and no collection was possible at all. There were microalgae on the bottom surface and the side surface of the culture container. However, these were not considered as subjects to be collected.

### [Example 6]

### <Temperature Dependence of Liquid Surface-floating Culture>

Pre-culture of AVFF007 strains of microalgae was performed using the same method as that in Example 1.

About 3 mL of a suspended solution of AVFF007 strains was prepared by collecting the AVFF007 strains on a liquid surface of a 6 hole plate and putting the total quantity of the AVFF007 strains into a tube for homogenizing with a 5 mL capacity using the same method as that in Example 1.

A solution having a concentration (initial algal body concentration) of the AVFF007 strains of 10 x 10⁴ cells/mL was prepared by adding 50 mL of a CSiFF01 medium shown in Fig. 23 and the suspended solution of the AVFF007 strains of the algae to a centrifugal settling tube with a 50 mL capacity. The suspended solution of the AVFF007 strains was well stirred and was put into a 6 hole plate at 8 mL per hole. The plate was put into a vacuum desiccator to perform culturing.

Room temperature culture and variable temperature culture were simultaneously performed at temperatures for each culturing.

For the room temperature culture, the 6 hole plate which was put into the vacuum desiccator was placed in a plant bioshelf for tissue culture, the CO₂ concentration was set to 5%, and the vacuum desiccator was locked with a lid to start liquid surface-floating culture. The culture conditions for the liquid surface-floating culture through stationary culture were 4000 lux for the amount of light (12 hours on-off control) and 23°C for the temperature.

For the variable temperature culture, the 6 hole plate which was put into the vacuum desiccator was provided in a shaking incubator (RGS-20RL, Sanki Seiki Co., Ltd.), the CO₂ concentration was set to 5%, and the vacuum desiccator was locked with a lid to start liquid surface-floating culture. The culture conditions for the liquid surface-floating culture through stationary culture were 4000 lux (continuous light) for the amount of light and each of set temperatures (specifically 20°C, 25°C, 30°C, 35°C, 37°C, 38°C, 39°C, and 40°C).

The liquid surface-floating culture was performed for 7 days. The collection of the biofilm on the liquid surface was performed using the same method as that of the collecting method using the first substrate (collecting method through transfer) of Fig. 11. The measurement of the quantity of the dry alga bodies of the biofilm was performed using the method in Example 2.

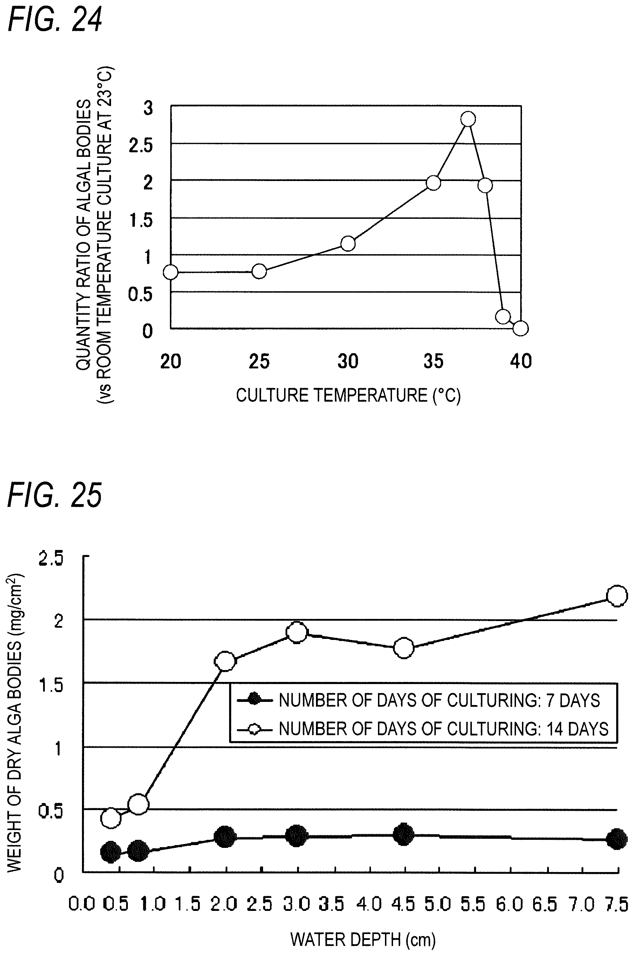

The relationship between the temperature for liquid surface-floating culture and the quantity ratio of dry alga bodies (quantity of dry alga bodies of the biofilm when culturing at room temperature of 23°C was regarded as a standard) in a biofilm at each temperature for liquid surface-floating culture is shown in Fig. 24. The higher the temperature was, the higher the quantity ratio of the dry alga bodies was until the temperature for the liquid surface-floating culture was 37°C. The quantity ratio of the dry alga bodies at 37°C was about 3 times that at room temperature (23°C). However, a biofilm was not formed on the liquid surface at 40°C. From the above, it can be seen that it is preferable to perform proliferation at a culture temperature of lower than or equal to 38°C in the case of the liquid surface-floating culture using the AVFF007 strains.

### [Example 7]

### <Influence of Water Depth of Medium on Liquid Surface-floating Culture>

A suspended solution of AVFF007 strains with a concentration of algal bodies of the AVFF007 strains of 9726 x 10⁴ cells/mL was prepared similarly to Example 1.

CSiFF01 medium was added respectively to tall Petri dishes having heights of 45 mm, 60 mm, and 90 mm (part No. 1-4402, As One Corporation) in the following quantities and media having different water depths were prepared.
Tall Petri dish with height of 45 mm: 9.5 mL (water depth: 0.4 cm), 19 mL (water depth: 0.8 cm), 47.5 mL (water depth: 2 cm), and 71.3 mL (water depth: 3 cm)
Tall Petri dish with height of 60 mm: 107 mL (water depth: 4.5 cm)
Tall Petri dish with height of 90 mm: 178 mL (water depth: 7.5 cm)

The suspended solution of the AVFF007 strains was added to glass tall Petri dishes at 195 µL per each dish and the dishes were stirred (that is, stirred while maintaining a constant number of added algal bodies of AVFF007 strains). In addition, the total number of the algal bodies in each of the tall Petri dishes was the same as in each of the others since the added amounts of the suspended solution of the AVFF007 strains were the same as each other while the liquid quantity of the medium varied in each dish. However, the concentration of the algal bodies varied in each dish, and therefore, the deeper the water depth was, the lower the concentration of the algal bodies was. The tall Petri dishes were put into a vacuum desiccator without being covered with lids. The CO₂ concentration was set to 5%.

Next, the tall Petri dishes containing the suspended solution of the AVFF007 strains were moved to the plant bioshelf for tissue culture and were subjected to culturing under light irradiation using a fluorescent lamp at 4000 lux. The room temperature was set to 23°C and liquid surface-floating culture through stationary culture was performed. Light was provided by performing 12 hour on-off control. The numbers of days of the culturing were 7 days and 14 days.

The collection of the biofilm on the liquid surface was performed by the same method as that of Example 3.

The relationship between the water depth of the media and the quantity of the dry alga bodies of the biofilms at each water depth of the media is shown in Fig. 25. In the drawing, the white circles are the results for the numbers of days of culturing of 7 days and the black circles are the results for the number of days of culturing of 14 days. The deeper the water depth of the medium was, the larger the quantity of the dry alga bodies of the biofilm on the liquid surface was. When the water depth increased from 0.8 cm to 2 cm, the quantity of the dry alga bodies steeply increased. However, even when the water depth increased further thereafter, the quantity of the dry alga bodies slightly increased.

From the above, it was found that, in the liquid surface-floating culture using the AVFF007 strains, if the water depth of the medium is greater than or equal to 0.4 cm, it is possible to culture the strains, and if the water depth thereof is deeper than or equal to 2 cm, the proliferation rate becomes higher and the quantity of the collected dry alga bodies of the biofilm increases.

### [Example 8]

### <Influence of Amount of Light, Concentration of Medium, and Number of Days of Culturing on Liquid Surface-floating Culture>

A suspended solution of AVFF007 strains having a concentration of algal bodies of AVFF007 strains of 16.7 x 10⁴ cells/mL was prepared similarly to Example 1.

2x CSiFF01 medium and 5x CSiFF01 medium were respectively prepared in addition to the CSiFF01 medium (1000 mL) shown in Fig. 23 as the liquid media. The 2x CSiFF01 medium and the 5x CSiFF01 medium respectively mean that the concentrations thereof are twice the 1x CSiFF01 medium and 5 times the 1x CSiFF01 medium.

After the preparation, an autoclave treatment was performed for 10 minutes at 121°C. 200 mL of the above prepared suspended solution of the AVFF007 strains having a concentration of 16.7 x 10⁴ cells/mL was put into a 6 hole plate at 8 mL per well. Two wells were used for each test condition and the wells had 54 holes in total. The 6 hole plate was put into a vacuum desiccator without being covered with a lid to start the culturing at a CO₂ concentration of 5%.

Next, the plate was moved to a plant bioshelf for tissue culture and the culturing was performed under light irradiation using a fluorescent lamp at 4000 lux, 8000 lux, or 16000 lux. The temperature was set to 23°C and liquid surface-floating culture through stationary culture was performed. Light was provided by performing 12 hour on-off control.

The collection of the biofilm on the liquid surface was performed using the same method as that of Example 3.

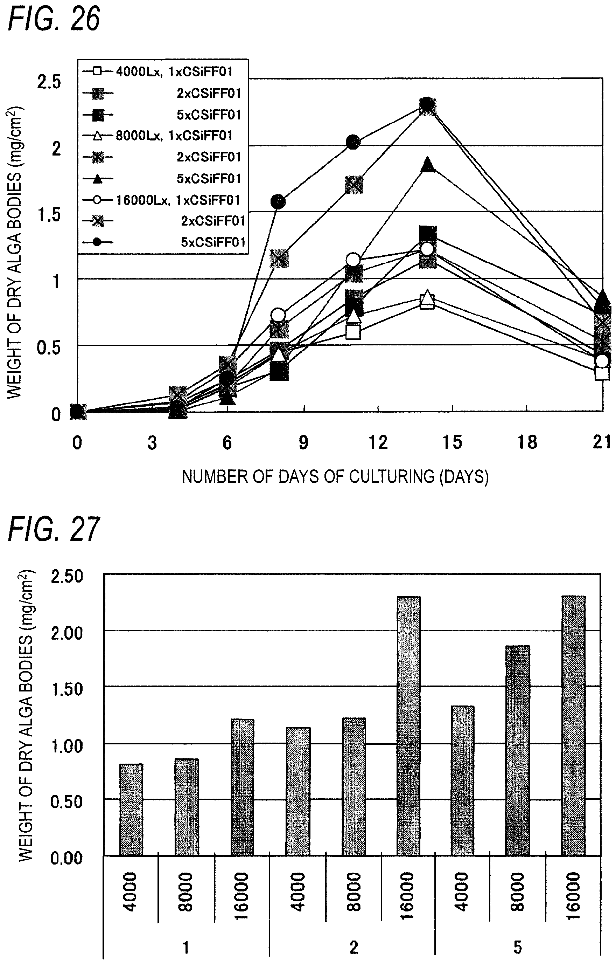

The relationship between the number of days of culturing in each concentration of a medium and the amount of light, and the quantity of dry alga bodies of a biofilm is shown in Fig. 26. In addition, in the number of days of culturing of 14 days, the relationship between the amount of light in each concentration of a medium and the quantity of dry alga bodies of a biofilm is shown in Fig. 27. The numerical values 1, 2, and 5 on the horizontal axis in Fig. 27 mean the results when respectively using the 1x CSiFF01 medium, the 2x CSiFF01 medium, and the 5x CSiFF01 medium and the numerical values described thereon mean the amount of light. There were tendencies that the larger the amount of light was and the higher the concentration of the medium was, the higher the proliferation rate was and the greater the quantity of the collected dry alga bodies of the biofilm was.

### [Example 9]

### <Influence of Calcium on Liquid Surface-floating Culture>

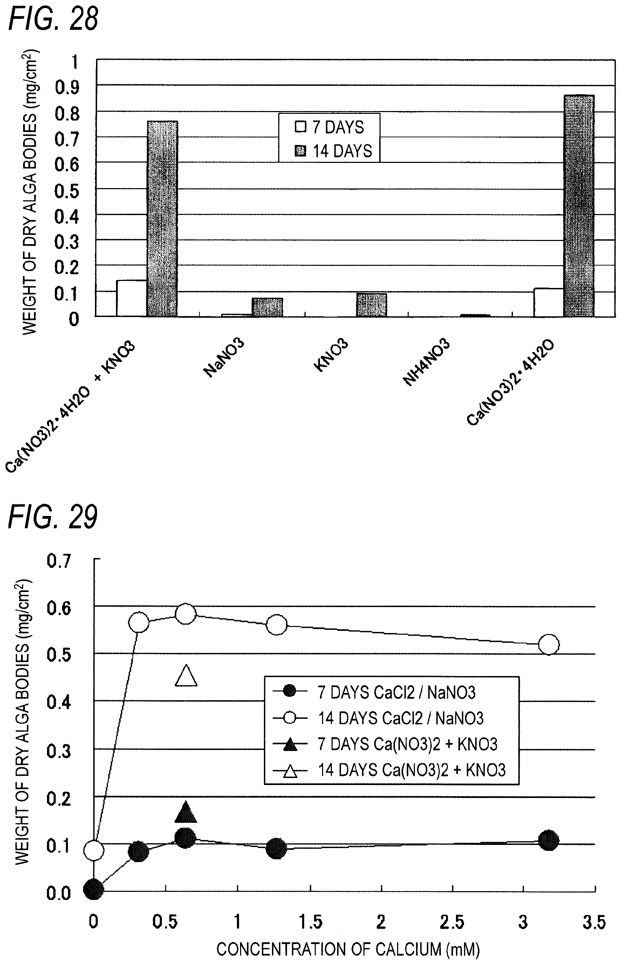

Four kinds of media in which nitrate sources (specifically, Ca(NO₃)₂·4H₂O and KNO₃ in CSiFF01 medium) in the CSiFF01 medium (1000 mL) shown in Fig. 23 were changed so as to be equimolar with those of various nitrate sources shown in Fig. 28 were prepared.

50 mL of a suspended solution of AVFF007 strains having a concentration of algal bodies of AVFF007 strains of 10 x 10⁴ cells/mL was prepared similarly to Example 1. The solution was put into the wells of a 6 hole plate at 8 mL per well to start liquid surface-floating culture through stationary culture. The 6 hole plate was put into a vacuum desiccator without being covered with a lid to start the culturing such that 5% CO₂ gas within the vacuum desiccator was reached. The amount of light was 4000 lux. The numbers of days of culturing performed were 7 days and 14 days.

The collection of the biofilm on the liquid surface was performed using the same method as that of Example 3.

The relationship between various nitrate sources and the quantity of dry alga bodies of the biofilm formed on the liquid surface for the numbers of days of culturing of 7 days and 14 days is shown in Fig. 28. The nitrate source of the standard CSiFF01 medium is a mixture of Ca(NO₃)₂·4H₂O and KNO₃. The quantity of the dry alga bodies after 14 days greatly increased when Ca(NO₃)₂·4H₂O was contained in the nitrate sources compared to when NaNO₃, KNO₃, and NH₄NO₃ were contained therein. The results show that the existence of calcium sources in a medium is important for the liquid surface-floating culture.

### [Example 10]

### <Influence of Calcium Concentration on Proliferation Rate>

Five kinds of media which had different concentrations of calcium were prepared such that there was 0 mM, 0.32 mM, 0.64 mM, 1.27 mM, and 3.18 mM of CaCl₂·2H₂O with respect to media to which Ca(NO₃)₂·4H₂O was not added in the CSiFF01 medium shown in Fig. 23.

A suspended solution of AVFF007 strains having a concentration of algal bodies of AVFF007 strains of 10 x 10⁴ cells/mL was prepared similarly to Example 1. The solution was put into the wells of a 6 hole plate at 8 mL per well to start liquid surface-floating culture through stationary culture. The 6 hole plate was put into a vacuum desiccator without being covered with a lid to start the culturing such that 5% CO₂ gas within the vacuum desiccator was reached. The amount of light was 4000 lux. The numbers of days of culturing performed were 7 days and 14 days.

The collection of the biofilm on the liquid surface was performed using the same method as that of Example 3.

The relationship between the concentration of calcium and the quantity of dry alga bodies of the biofilm formed on the liquid surface for the numbers of days of culturing of 7 days and 14 days is shown in Fig. 29. The formation of the biofilm formed of the microalgae on the liquid surface could be seen at a concentration of calcium of higher than or equal to 0.32 mM whereas there was little quantity of the algal bodies of the microalgae on the liquid surface when the concentration of calcium was 0. These results show that the existence of calcium is important for forming the biofilm on the liquid surface.

### [Example 11]

### <Influence of pH on Liquid Surface-floating Culture>

A suspended solution of AVFF007 strains having a concentration of algal bodies of AVFF007 strains of 19713 x 10⁴ cells/mL was prepared similarly to Example 1.

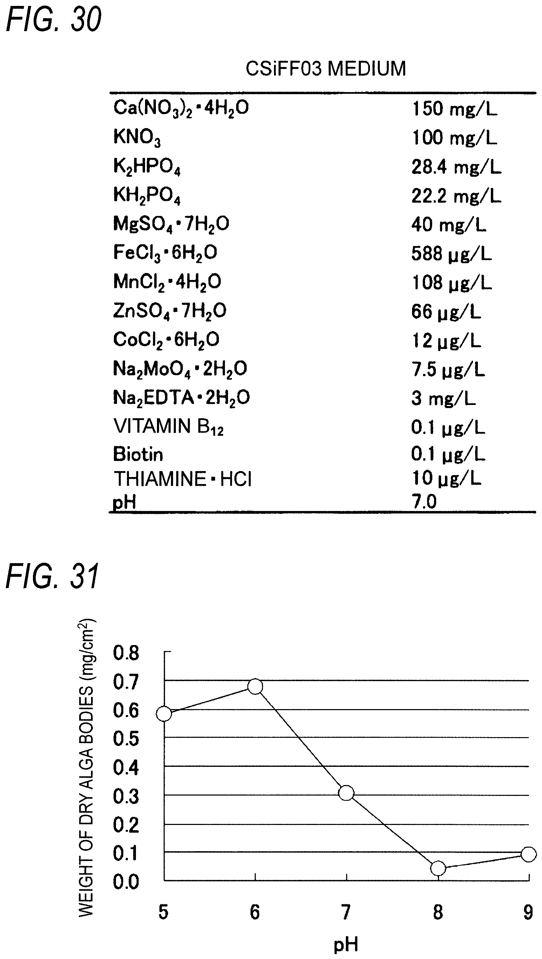

50 mL of respective media, in which the pH of a CSiFF03 medium shown in Fig. 30 was adjusted to 5 to 9, were put into a centrifugal settling tube with a 50 mL capacity and 25.4 µL of the suspended solution of the AVFF007 strains was added thereto to prepare a suspended solution of the AVFF007 strains having an initial algal body concentration of 10 x 10⁴ cells/mL. The suspended solution of the AVFF007 strains was well stirred and was put into a 6 hole plate at 8 mL per hole.

The 6 hole plate was put into a vacuum desiccator and was placed in a plant bioshelf for tissue culture. The vacuum desiccator was locked with a lid to start culture under a 5% CO₂ atmosphere. The culture conditions for the liquid surface-floating culture through stationary culture were 4000 lux for the amount of light (12 hours on-off control) and 23°C for the temperature.

The culturing was performed for 14 days. The collection of the biofilm was performed using the same method as that of Example 3. The measurement of the quantity of the dry alga bodies was performed using the method in Example 2.

The relationship between the pH and the quantity of the dry alga bodies of a biofilm formed on a liquid surface is shown in Fig. 31. In the culturing method of the related art, the culturing was generally performed at pH 7. However, it was found that a lower pH enabled favorable culturing.

From the above, it was found that it is preferable that the pH of the medium of the liquid surface-floating culture be less than or equal to 7 when using the AVFF007 strains.

### [Example 12]

### <Influence of Carbon Dioxide in Air (Gas Phase) on Liquid Surface-floating Culture>

A solution containing AVFF007 strains on which microorganism purification was performed was put into a conical flask with a 100 mL capacity into which 40 mL of a mixed medium of C medium and CSi medium (volume ratio of 1:1) was put, the conical flask was placed in a plant bioshelf for tissue culture, and culturing was performed at 23°C under continuous light irradiation of 4000 lux. A conical flask with a 100 mL capacity sterilized by dry heat for 10 minutes at 180°C in advance was used. In addition, the culturing performed was stationary culture.

A biofilm on a liquid surface was collected using the same method as that of Example 4 except for using a polyethylene film instead of Parafilm and a suspended solution of microalgae was prepared using the same method as that of Example 1. The count number of algal bodies was 13900 x 10⁴ cells/mL. Therefore, 179.9 µL of the suspended solution of the microalgae was used to prepare 250 mL of a dispersed solution of microalgae having a concentration of 10 x 10⁴ cells/mL.

The suspended solution of AVFF007 strains which had a concentration of 10 x 10⁴ cells/mL and was prepared above was put into a 6 hole plate at 8 mL per well. Two wells were used for each test condition and the wells had 30 holes in total. The 6 hole plate was covered with a lid.

Next, the 6 hole plate containing the suspended solution of the microalgae was moved to a plant bioshelf for tissue culture to perform culturing under light irradiation using a fluorescent lamp at 4000 lux. The room temperature was set to 23°C and liquid surface-floating culture through stationary culture was performed. Light was provided by performing 12 hour on-off control. In addition, in order to perform the culturing under a CO₂ atmosphere, a 6 hole plate into which a CO₂-generating agent and the above-described microalgae were put was provided in a vacuum desiccator and CO₂ was generated so as to obtain a target CO₂ concentration. The culturing started after covering the vacuum desiccator with a lid. The period of culturing performed was 7 days, 14 days, or 21 days.

The collection of the biofilm on the liquid surface was performed using the same method as that of Example 4 except for using the polyethylene film instead of Parafilm and the same method as that of Example 1 was performed to count the number of algal bodies.

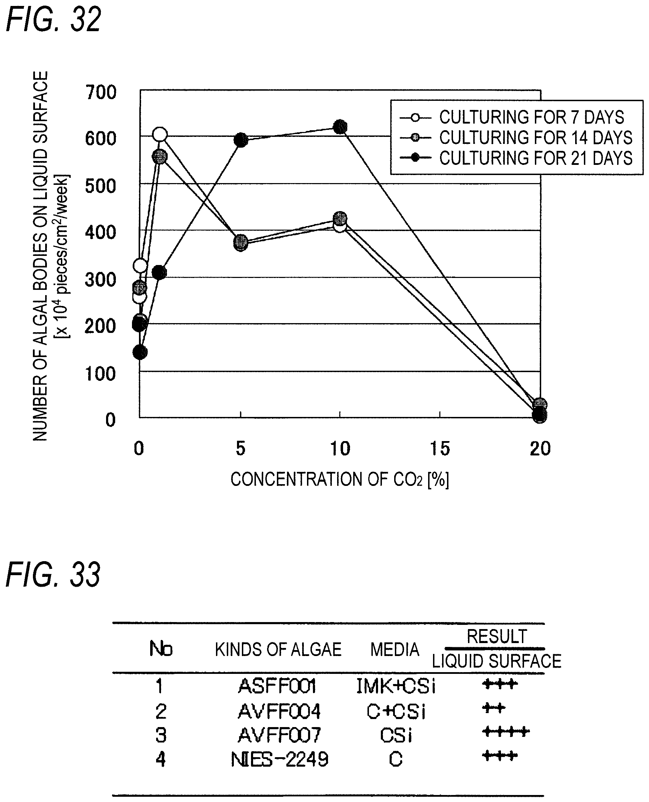

The relationship between the CO₂ concentration (volume%) in the gas phase and the number of algal bodies of the biofilm formed on the liquid surface is shown in Fig. 32.

As described above, it was found that it was possible to perform the culturing at a CO₂ concentration higher than or equal to the CO₂ concentration in the air and less than 20 volume% in the liquid surface-floating culture using the AVFF007 strains.

A CO₂ concentration of 0 volume% refers to a case in which the main body of the 6 hole plate and the lid were sealed with Parafilm. Meanwhile, a CO₂ concentration of 0.04 volume% is the CO₂ concentration in the air and refers to a case in which the main body of the 6 hole plate and the lid were not sealed with Parafilm. In addition, the culturing for both the cases was performed in the vacuum desiccator, but no CO₂ generator was used for both the cases.

In addition, in regards to the CO₂ concentration in the present example, it is considered that since CO₂ was not bubbled in the medium, the AVFF007 strains proliferated using CO₂ in the air.

### [Example 13]

### <Liquid Surface-floating Culture of Other Microalgae>

Microalgae, with which it is possible to perform liquid surface-floating culture, except for AVFF007 strains were searched for using four kinds of microalgae and media shown in Fig. 33. In a case of using a mixed medium shown in Fig. 33, media were mixed together at a liquid amount ratio of 1:1.

Respective subcultured microalgae were put into a clean bench and were allowed to stand for a while in order to allow the microalgae in a conical flask to sink to the bottom surface thereof. In the subculturing, the majority of all of the microalgae did not exist on the liquid surface of the conical flask. After the microalgae had sunk to the bottom surface of the conical flask, a solution containing the microalgae was collected using a pipette, the solution was put into a tube for homogenizing with a 5 mL capacity (TM-655S) for setting in a beads cell disrupter (MS-100), and then, a homogenization treatment lasting for 20 seconds was performed 3 times at 4200 rpm.

Next, 20 mL of a medium shown in Fig. 33 was put into a centrifugal settling tube with a 50 mL capacity and a suspended solution of microalgae for culture was prepared by adding the suspended solution of the microalgae described above.

The above-described solution was stirred, and then, was put into a 6 hole plate at 8 mL per well. Two wells were used for each test condition.

Next, the 6 hole plate containing the suspended liquid of the microalgae was moved to a plant bioshelf for tissue culture and was put into a vacuum desiccator to perform the culturing for 7 days under light irradiation using a fluorescent lamp at 4000 lux. The room temperature was set to 23°C and the culturing performed was stationary culture. Light irradiation was performed by being turned on and off at a time interval of 12 hours.

Whether the microalgae were cultured on the liquid surface was determined by observation using a microscope (magnification of 4).

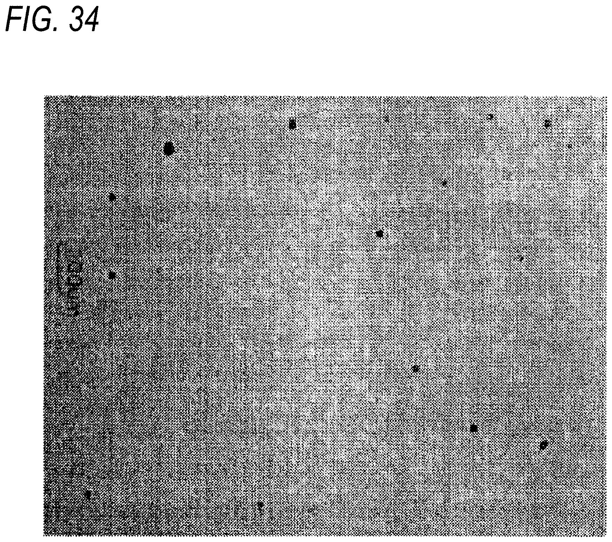

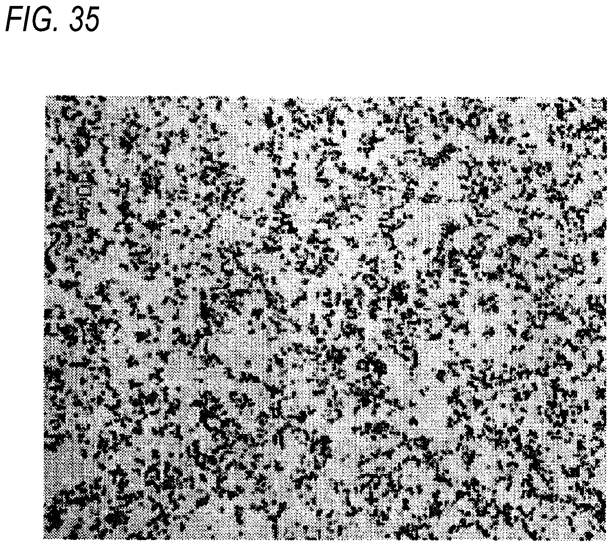

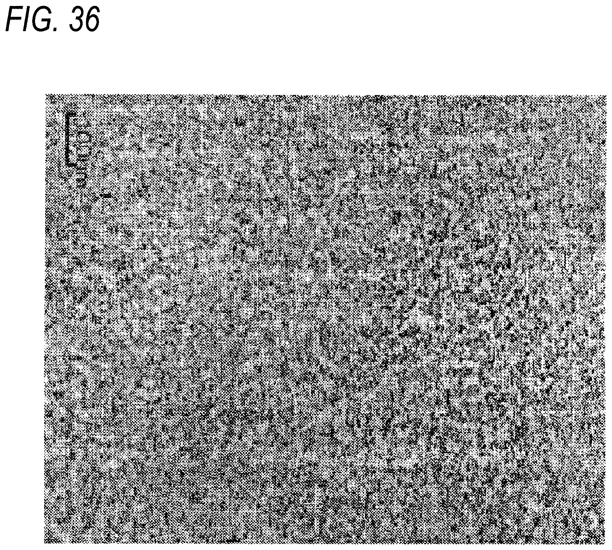

In Fig. 33, + indicates a case in which the microalgae barely existed on the liquid surface, ++ indicates a case in which a few microalgae existed on the liquid surface, +++ indicates a case in which a fair number of microalgae existed on the liquid surface, and ++++ indicates a case in which a clear biofilm formed on the liquid surface. The example of AVFF004 strains is shown in Fig. 34 as a representative example of ++, the example of NIES-2249 strains is shown in Fig. 35 as a representative example of +++, and the example of AVFF007 strains is shown in Fig. 36 as a representative example of ++++.

The density of the microalgae on the liquid surface per unit area was measured by directly performing counting using the microphotograph. The results are shown below together with the above-described evaluation of + to ++++.
ASFF001 strains: 630,000 pieces/cm² (+++)
AVFF004 strains: 1,400 pieces/cm² (++)
AVFF007 strains: 1,000,000 pieces/cm² (++++)
NIES-2249 strains: 500,000 pieces/cm² (+++)

The NIES-2249 strains are Chlorococcum echinozygotum Starr purchased at the National Institute of Environmental Studies. An AS series and an AV series were respectively collected from fresh water in Shizuoka-ken and fresh water in Kyoto and were subjected to microorganism purification.

In the present invention, it is considered that the microalgae determined as ++ and +++ can form a clear biofilm on a liquid surface like ++++ by devising the culture conditions based on the findings disclosed in the present application and by creating preferable culture conditions.

### [Example 14]

### <Influence of Amount of Light on Liquid Surface-floating Culture>

Microalgae were collected by transferring AVFF007 strains which were pre-cultured on a 6 hole plate using the CSiFF03 medium shown in Fig. 30 using a first substrate. The total quantity of microalgae was added to a 5 mL tube for homogenizing containing the CSiFF03 medium and the turbidity in the tube was measured through a high speed-shaking treatment using a spectrophotometer. As a result, it was determined that the concentration of the algal bodies was 19816 x 10⁴ pieces.

A solution of 50 x 10⁴ cells/mL was prepared by adding 131 µL of a dispersed solution of the AVFF007 strains to a container into which 52 mL of the CSiFF04 medium shown in Fig. 16 was put in advance and by stirring the mixture well.

The solution was put into a 23-type case made of polystyrene (4-5605-04, external size of 30 mm x 30 mm, As One Corporation) at 5 mL in each, and 10 cases thereof were prepared.

Lumina Ace (1-7373-01, halogen lamp, As One Corporation) was used as a light source and light in a range of 2600 lux to 90800 lux was radiated through on-off control at a 12 hours interval. After 14 days, a film-like structure on a liquid surface was collected using a second substrate. Other test conditions included 23°C for the temperature, stationary culture, the concentration of the air for the CO₂ concentration, and 0.8 cm for the water depth of the liquid medium. In addition, two sample containers were prepared for each amount of light. The amount of light was set to be equal as much as possible by adjusting the setting of the amount of light of the main body of the Lumina Ace and the distance between the light source and the sample.

The evaluation was performed by measuring the dry weight. The total quantity of the microalgae obtained using the second substrate, that is, a nylon film was transferred to the top of a cover glass, which was weighed in advance, using a cell scraper and the transferred microalgae were heat-dried for 30 minutes at 120°C. Then, the dry weight was calculated from the weight difference. An average value of two samples was used for the dry weight.

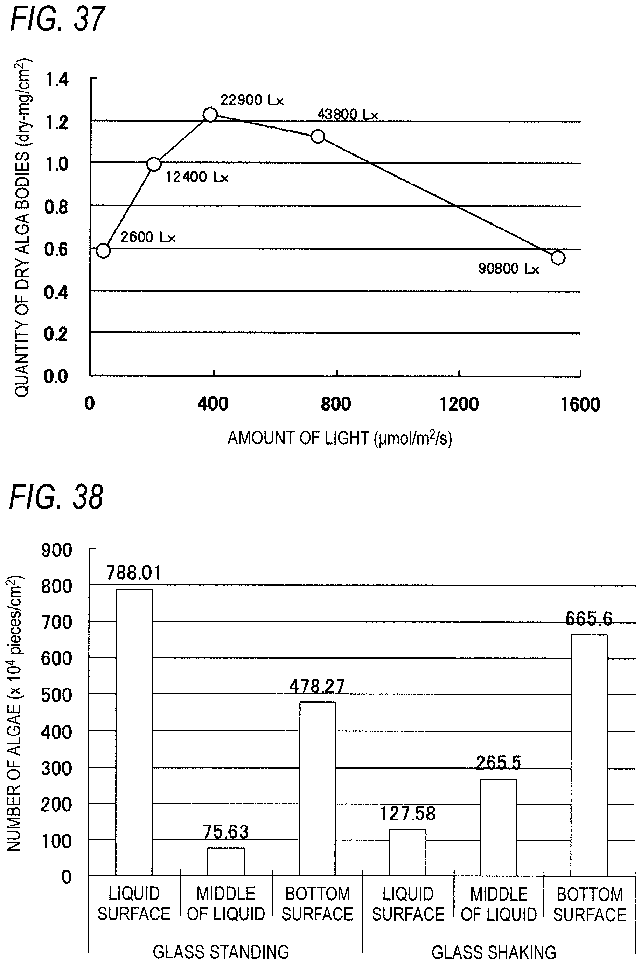

The results are shown in Fig. 37. Deterioration of proliferation rate, which is considered to be generally caused by light disorder, can be seen in the culturing of microalgae under a high amount of light such as 100,000 lux corresponding to the light amount of sunlight in fine weather, and in some cases, microalgae do not proliferate depending on the kinds of microalgae. It was shown that the maximum proliferation rate of the AVFF007 strains was at 22900 lux and that it is also possible to secure approximately half the maximum proliferation rate even at 90800 lux, which enables culturing under the high amount of light.

### [Example 15]

### [Stationary Culture and Shaking Culture]

A suspended liquid having an alga body concentration of 1 x 10⁴ cells/mL was prepared using the same method as that of Example 12. The count number of algal bodies was 3350 x 10⁴ cells/mL. Therefore, 507.5 µL of the dispersed solution of microalgae was used to prepare 170 mL of a dispersed solution of microalgae having a concentration of 10 x 10⁴ cells/mL.

The dispersed solution with a concentration of 10 x 10⁴ cells/mL of microalgae which was prepared above was put into 4 conical glass flasks with a 100 mL capacity at 20 mL for each. Two conical flasks were used for each test condition. The conical flask was covered with a plug using Plug Silicon (As One Corporation).

When performing stationary culture, the conical flask containing the suspended liquid of the microalgae was moved to a plant bioshelf for tissue culture to perform the culturing under light irradiation using a fluorescent lamp at 4000 lux. The room temperature was set to 23°C. The light was continuous light.

When performing the shaking culture, the conical flask containing the suspended liquid of the microalgae was moved to a shaking incubator (RGS-20RL, Sanki Seiki Co., Ltd.) to perform the culturing at a shaking rate of 100 rpm under light irradiation using a fluorescent lamp at 4000 lux. The temperature in the culture container was set to 23°C. The light was continuous light.

The culture period was 1 week for both the cases.

The collection of the microalgae on the liquid surface was performed using the same method as that of Example 4. The collection of the microalgae in the middle of the liquid was performed by collecting media in the vicinity of the midway between the liquid surface and the bottom surface using a pipette after collecting the microalgae on the liquid surface. The number of microalgae was counted using the same method as that of Example 1. The collecting and the counting of the microalgae on the bottom surface were performed such that after collecting the microalgae on the liquid surface, the medium was removed and a new medium was added and dispersed. Then, the number of microalgae was counted using the same method as that of Example 1. However, polyethylene was used instead of the Parafilm.

The results are shown in Fig. 38. The number of algal bodies on the liquid surface was 788 x 10⁴ cells/mL when the stationary culture was performed and 127 x 10⁴ cells/mL when the shaking culture was performed. The number of algal bodies when the stationary culture was performed was significantly high. Accordingly, in the present invention, it is preferable to perform the stationary culture. However, the biofilm of the algal bodies was formed on the liquid surface even when the shaking culture was performed, and therefore, the shaking culture may also be performed. The biofilm on the liquid surface is clearly visible even in the case of the culture container of this test. In the case of the shaking culture, the number of microalgae in the middle of the liquid and on the bottom surface is greater than that of the stationary culture. In addition, it is considered that the proportion of the number of microalgae in the middle of the liquid and on the bottom surface changes depending on the time from the stoppage of the shaking to the collection of the sample for counting.

In addition, it is considered that, in the stationary culture, from the results, the significant increase in the number of algal bodies was influenced by use of CO₂ in the air using the Plug Silicon and the material of the culture container.

As is clear from Fig. 38, the majority of microalgae existed on the liquid surface and the bottom surface when the stationary culture was performed.

In regards to the proliferation of Microcystis, it is known that a fair number thereof exist even at the water depth of about 3 m from the surface of water, and it is obvious that the proliferation of the Microcystis is different from the proliferation of the microalgae in the stationary culture of the present invention.

### [Comparative Example 1]

### <Culturing Using NIES-2199 Strains>

Culturing was performed by the following method using NIES-2199 strains purchased at the National Institute of Environmental Studies. According to the data of the National Institute of Environmental Studies, the NIES-2199 strains are Botryococcus braunii Kutzing.

It is described that the NIES-2199 Strains contain contaminative microorganisms. Therefore, the culturing of the NIES-2199 strains was performed on agarose gel containing a C medium, and a proliferated colony was seeded to a well in a 24 hole plate into which 2 mL of the C medium was put in advance to perform microorganism purification and pre-culturing.

The plate was placed in a plant bioshelf for tissue culture and was subjected to stationary culture for 20 days at 4000 lux at room temperature under the conditions in which turning on and off the light irradiation was performed every 12 hours. However, the NIES-2199 strains did not float on the liquid surface having sunken to the bottom surface of the medium.

### Industrial Applicability

It is possible to form a biofilm on a liquid surface using the method for culturing microalgae according to the present invention and it is possible to greatly reduce the collecting cost compared to that of the collection of microalgae in the related art. In addition, it is possible to collect the biofilm formed on the liquid surface at a high collection rate and the water content ratio thereof is low. Furthermore, it is possible to obtain oil from the collected biofilm and the biofilm can also be expected to be biomass intended for prevention of global warming.

In addition, the microalgae according to the present invention can form a biofilm on a liquid surface and it is possible to greatly improve the collection efficiency and to greatly reduce the collecting cost compared to those of the collection of microalgae in the related art. In addition, the water content ratio of the collected biofilm is the same as that of the method of the related art, or lower than or equal to that of the method of the related art, thereby greatly reducing the cost in a dehydration process. In addition, it is possible to obtain oil from the collected biofilm and the microalgae can also be expected to be biomass intended for prevention of global warming.

The present invention was described in detail or with reference to specific embodiments, but is defined in the claims.

## Claims

1. A method for culturing microalgae, comprising:
culturing microalgae capable of forming a biofilm on a liquid surface such that the biofilm is formed on a liquid surface of a liquid medium, wherein
(i) the homology of the microalgae with base sequences of a region having greater than or equal to 1000 bases corresponding to *Botryococcus sudeticus* 18S rRNA sequence SEQ ID NO:1 is 95.0% to 99.9%; or
(ii) the microalgae are *Botryococcus sudeticus* AVFF007 strains (deposition number FERM BP-11420).

2. The method for culturing microalgae according to claim 1, further comprising:
performing stationary culture of microalgae capable of forming the biofilm on the liquid surface.

3. The method for culturing microalgae according to claim 1 or 2,
wherein the liquid medium contains calcium.

4. The method for culturing microalgae according to claim 3,
wherein the concentration of calcium in the liquid medium is higher than or equal to 0.3 mM.

5. The method for culturing microalgae according to any one of claims 1 to 4,
wherein a concentration of carbon dioxide in a gas phase is greater than or equal to a concentration of carbon dioxide in the air and less than 20 volume%.

6. A biofilm formed on a liquid surface by the method for culturing the microalgae according to any one of claims 1 to 5.

7. Microalgae capable of forming a biofilm on a liquid surface, wherein
(i) the homology of the microalgae with base sequences of a region having greater than or equal to 1000 bases corresponding to Botryococcus sudeticus 18S rRNA sequence SEQ ID NO:1 is 95.0% to 99.9%; or
(ii) the microalgae are *Botryococcus sudeticus* AVFF007 strains (deposition number FERM BP-11420).

8. The microalgae according to claim 7,
wherein the microalgae are *Botryococcus sudeticus* AVFF007 strains (deposition number of FERM BP-11420).

9. A method for collecting a biofilm,
wherein the biofilm formed on the liquid surface according to claim 6 is collected by depositing the biofilm on a substrate.

10. A method for collecting a biofilm,
wherein the biofilm formed on the liquid surface according to claim 6 is collected by transferring the biofilm onto a substrate.

11. A method for producing biomass fuel,
wherein the biofilm collected by the method for collecting the biofilm according to any one of claims 9 or 10 is used as fuel.

## Patentansprüche

1. Verfahren zur Kultivierung von Mikroalgen, umfassend:
Kultivieren von Mikroalgen, die in der Lage sind, einen Biofilm auf einer Flüssigkeitsoberfläche zu bilden, so dass der Biofilm auf einer Flüssigkeitsoberfläche eines flüssigen Mediums gebildet wird, worin
(i) die Homologie der Mikroalgen mit Basensequenzen eines Bereichs mit mehr als oder gleich zu 1.000 Basen, entsprechend Botryococcus sudeticus 18S rRNA-Sequenz SEQ ID NO: 1, 95,0 % bis 99,9 % beträgt; oder
(ii) die Mikroalgen Botryococcus sudeticus AVFF007-Stämme sind (Hinterlegungsnummer FERM BP-11420).

2. Verfahren zum Kultivieren von Mikroalgen gemäß Anspruch 1, ferner umfassend:
Durchführen einer stationären Kultur der Mikroalgen, die in der Lage sind, den Biofilm auf der Flüssigkeitsoberfläche zu bilden.

3. Verfahren zum Kultivieren von Mikroalgen gemäß Anspruch 1 oder 2, worin das flüssige Medium Calcium enthält.

4. Verfahren zum Kultivieren von Mikroalgen gemäß Anspruch 3, worin die Konzentration von Calcium in dem flüssigen Medium größer als oder gleich zu 0,3 mM ist.

5. Verfahren zum Kultivieren von Mikroalgen gemäß irgendeinem der Ansprüche 1 bis 4, worin die Konzentration von Kohlendioxid in der Gasphase größer als oder gleich zur Konzentration von Kohlendioxid in der Luft und weniger als 20 Vol.% beträgt.

6. Biofilm, gebildet auf einer Flüssigkeitsoberfläche durch das Verfahren zum Kultivieren der Mikroalgen gemäß irgendeinem der Ansprüche 1 bis 5.

7. Mikroalgen, die in der Lage sind, einen Biofilm auf einer Flüssigkeitsoberfläche zu bilden, worin
(i) die Homologie der Mikroalgen mit Basensequenzen eines Bereichs mit mehr als oder gleich zu 1.000 Basen, entsprechend Botryococcus sudeticus 18S rRNA-Sequenz SEQ ID NO: 1, 95,0 % bis 99,9 % beträgt; oder
(ii) die Mikroalgen Botryococcus sudeticus AVFF007-Stämme (Hinterlegungsnummer FERM BP-11420) sind.

8. Mikroalgen gemäß Anspruch 7, worin die Mikroalgen Botryococcus sudeticus AVFF007-Stämme (Hinterlegungsnummer FERM BP-11420) sind.

9. Verfahren zum Sammeln eines Biofilms,
worin der Biofilm, der gemäß Anspruch 6 auf der Flüssigkeitsoberfläche gebildet ist, durch Abscheiden des Biofilms auf einem Substrat gesammelt wird.

10. Verfahren zum Sammeln eines Biofilms,
worin der Biofilm, gebildet auf der Flüssigkeitsoberfläche gemäß Anspruch 6, durch Transferieren des Biofilms auf ein Substrat gesammelt wird.

11. Verfahren zur Herstellung von Kraftstoff aus Biomasse, worin der durch das Verfahren zum Sammeln des Biofilms gemäß irgendeinem der Ansprüche 9 oder 10 gesammelte Biofilm als Kraftstoff verwendet wird.

## Revendications

1. Procédé de culture de micro-algues, comprenant :
la culture de micro-algues capables de former un biofilm sur la surface d'un liquide de telle sorte que le biofilm se forme sur la surface d'un liquide d'un milieu liquide, dans lequel
(i) l'homologie des micro-algues avec les séquences de bases d'une région ayant 1000 bases ou plus correspondant à la séquence d'ARNr 18S de *Botryococcus sudeticus* SEQ ID NO : 1 est de 95,0 % à 99,9 % ; ou
(ii) les micro-algues sont les souches AVFF007 de *Botryococcus sudeticus* (numéro de dépôt FERM BP-11420).

2. Procédé de culture de micro-algues selon la revendication 1, comprenant en outre :
la réalisation d'une culture stationnaire de micro-algues capables de former le biofilm sur la surface d'un liquide.

3. Procédé de culture de micro-algues selon la revendication 1 ou 2,
dans lequel le milieu liquide contient du calcium.

4. Procédé de culture de micro-algues selon la revendication 3,
dans lequel la concentration du calcium dans le milieu liquide est supérieure ou égale à 0,3 mM.

5. Procédé de culture de micro-algues selon l'une quelconque des revendications 1 à 4,
dans lequel une concentration en dioxyde de carbone dans une phase gazeuse est supérieure ou égale à une concentration en dioxyde de carbone dans l'air et inférieure à 20 % en volume.

6. Biofilm formé sur une surface d'un liquide par le procédé de culture des micro-algues selon l'une quelconque des revendications 1 à 5.

7. Micro-algues capables de former un biofilm sur la surface d'un liquide, dans lesquelles
(i) l'homologie des micro-algues avec les séquences de bases d'une région ayant 1000 bases ou plus correspondant à la séquence d'ARNr 18S de *Botryococcus sudeticus* SEQ ID NO : 1 est de 95,0 % à 99,9 % ; ou
(ii) les micro-algues sont les souches AVFF007 de *Botryococcus sudeticus* (numéro de dépôt FERM BP-11420).

8. Micro-algues selon la revendication 7,
dans lesquelles les micro-algues sont les souches AVFF007 de *Botryococcus sudeticus* (numéro de dépôt FERM BP-11420).

9. Procédé de collecte d'un biofilm,
dans lequel le biofilm formé sur la surface d'un liquide selon la revendication 6 est collecté par dépôt du biofilm sur un substrat.

10. Procédé de collecte d'un biofilm,
dans lequel le biofilm formé sur la surface d'un liquide selon la revendication 6 est collecté par transfert du biofilm sur un substrat.

11. Procédé de production de combustible de biomasse,
dans lequel le biofilm collecté par le procédé de collecte du biofilm selon l'une quelconque des revendications 9 ou 10 est utilisé en tant que combustible.


### SEQUENCE LISTING

<110> FUJIFILM Corporation
<120> Method for culturing microalgae, biofilm formed on liquid level by the culturing method, biomass and oil content obtained from the biofilm, collection method of the biofilm and production method of biomass fuel
   Microalgae capable of forming biofilm on liquid level, biofilm formed on liquid level by the microalgae, and biomass and oil content obtained from the biofilm
<130> W104926
<150> JP 2012-099188
   <151> 2012-04-24
<150> JP 2012-099189
   <151> 2012-04-24
<160> 3
<170> PatentIn version 3.5
<210> 1
   <211> 1111
   <212> DNA
   <213> Botryococcus sudeticus
<400> 1
   cggtcgcttc ggtgagtact gctatggcct tcctttctac gggggacggg ctcctgggat        60
   taacttctcg ggatccggat tccgtgatga tactttgagt aaattggagt gttcaaagca       120
   agcctgcgct ctgaacattt tagcatggaa tatcacgata ggactctggc ctatcttgtt       180
   ggtctgtagg accagagtaa tgattaagag ggacagtcgg gggcattcgt atttcattgt       240
   cagaggtgaa attcttggat ttatgaaaga cgaactactg cgaaagcatt tgccaaggat       300
   gttttcatta atcaagaacg aaagttgggg gctcgaagac gattagatac cgtcgtagtc       360
   tcaaccataa acgatgccga ctagggatta gcagacgttt cattgatgac tctgctagca       420
   ccttatgtga aaacaaagtt tttgggttcc ggggggagta tggtcgcaag gctgaaactt      480
   aaaggaattg acggaagggc accaccaggc gtggagcctg cggcttaatt tgactcaaca       540
   cgggaaaact taccaggtcc agacatagtg aggattgaca gattgagagc tctttcttga       600
   ttctatgggt ggtggtgcat ggccgttctt agttggtggg ttgccttgtc aggttgattc       660
   cggtaacgaa cgagacctca gctcctaaat agtccatgcc gccttttggc ggtatgctga       720
   cttcttagag ggactattgg cgttctagcc aatggaagta tgaggcaata acaggtctgt       780
   gatgccctta gatgttctgg gccgcacgcg cgctacactg atgcgttcaa cgagcctatc       840
   cttgaccgag aggtccgggt aatctttgaa accgcatcgt gatggggcta gactattgca       900
   attattagtc tttaacgagg aatgcctagt aagcgcgatt catcagatcg cgttgattac       960
   gtccctggta cacaccgccc gtcgctccta ccgattgggt gtgctggtga agtgttcgga      1020
   tcggcaaagc tgggggcaac tcttgcattg ccgagaagtt cattaaaccc tcccacctag      1080
   aggaaggaga agtcgtaaca aggtttccgt a                                     1111
<210> 2
   <211> 1727
   <212> DNA
   <213> Chlorella saccharophila
<400> 2
   tctcaagatt aaccatgcat gtctaagtat aaactgctta tactgtgaaa ctgcgaatgg 60
   ctcattaaat cagttatagt ttatttgatg gtacctacta ctcggataac cgtagtaatt 120
   ctagagctaa tacgtgcgta aatcccgact cctggaaggg acgtatttat tagataaaag 180
   gccgaccgga ctccgtccga ctcgcggtga atcatgataa cttcacgaat cgcatggcct 240
   cgtgccggcg atgtttcatt caaatttctg ccctatcaac tttcgatggt aggatagagg 300
   cctaccatgg ttttgacggg tgacggagga ttagggttcg attccggaga gggagcctga 360
   gaaacggcta ccacatccaa ggaaggcagc aggcgcgcaa attacccaat cccgacacgg 420
   ggaggtagtg acaataaata acaataccgg gctttttcaa gtctggtaat tggaatgagt 480
   acaatctaaa tcccttaacg aggatcaatt ggagggcaag tctggtgcca gcagccgcgg 540
   taattccagc tccaatagcg tatatttaag ttgttgcagt taaaaagctc gtagttggat 600
   ttcgggcggg gcctgccggt ccgcctctgg tgtgcactgg catgggctcg cctcgctgtc 660
   ggggacgggc tcctgggctt aactgtccgg gactcggagt cgacgaggtt actttgagta 720
   aattagagtg ttcaaagcac gcctacgctc tgaatacgtt agcatggaat aacacgatag 780
   gactctggcc tatcttgttg gtctgtggga ccggagtaat gattaagagg gacggtcggg 840
   ggcattcgta tttcattgtc agaggtgaaa ttcttggatt tatgaaagac gaacttctgc 900
   gaaagcattt gccaaggatg ttttcattaa tcaagaacga aagttggggg ctcgaagacg 960
   attagatacc gtcctagtct caaccataaa cgatgccgac tagggatcgg cgggtgttct 1020
   ttcgatgacc ccgccggcac cttacgagaa atcaaagttt ttgggttccg gggggagtat 1080
   ggtcgcaagg ctgaaactta aaggaattga cggaagggca ccaccaggcg tggagcctgc 1140
   ggcttaattt gactcaacac gggaaaactt accaggtcca gacatagtga ggattgacag 1200
   attgagagct ctttcttgat tctatgggtg gtggtgcatg gccgttctta gttggtgggt 1260
   tgccttgtca ggttgattcc ggtaacgaac gagacctcag cctgctaaat agtcacgctc 1320
   ggcttctgcc gggcggcagc gcttcttaga gggactatcg gcgactagcc gatggaagtg 1380
   tgaggcaata acaggtctgt gatgccctta gatgttctgg gccgcacgcg cgctacactg 1440
   acgcaatcaa cgagcccagc cttggtcgag agacccgggt aatcttgtaa cctgcgtcgt 1500
   gatggggcta gactcttgca attattagtc ttcaacgagg aatgcctagt aagcgcgagt 1560
   catcagctcg cgttgattac gtccctgccc tttgtacaca ccgcccgtcg ctcctaccga 1620
   ttggatgtgc tggtgaagcc ctaggactgg cggcctttgg tggttctccg ccgacggtcg 1680
   ccgggaattc ggttaaaccc tcccatctag aggaggagaa ctcgtaa 1727
<210> 3
   <211> 1201
   <212> DNA
   <213> Makinoella tosaensis
<400> 3
   gattaagcat gcatgtctaa gtataagctg cttatactgt gaaactgcga atggctcatt      60
   aaatcagtta tagtttattt gatggtacct tactactcgg atacccgtag taattctaga     120
   gctaatacgt gcgcacatcc cgacttctgg aagggacgta tttattagat aaagaccgac     180
   cggctctgcc gaccctcggt gaatcatgat aacttcacga atcgcatggc ctcgtgccgg     240
   cgatgtttca ttcaaatttc tgccctatca actttcgatg gtaggataga ggcctaccat     300
   ggtgttaacg ggtgacggag aattagggtt cgattccgga gagggagcct gagaaacggc     360
   taccacatcc aaggaaggca ccaggcgccc aaattaccaa atcccgacac ggggaggtag     420
   tgacaaaaaa taacaatact tggctcattc gagtccggta attggaatga gtacaatcta     480
   aaccccttaa cgaggatcca ttggagggca agtctggtgc cagcagccgc ggtaattcca     540
   gctccaatag cgtatattta agttgctgca gttaaaaagc tcgtagtcgg ttctcgggag     600
   gggtggcacg gtccgccttg tgcgtgcact gcgccgcccc tctttaccgc tggtgaccag     660
   ctcctgggct tcgctgtccg ggactgggag tcagcactgt taccttgagt aaattggagt     720
   gttcaaagca agcaaacgct gtgcacactt tagcatggaa taacgatcat tggactcgcg     780
   tccagtcgtt ggtctatggt ctgcgagtaa cgaccaatgg gggcagtcgg gggcatttgt     840
   attgcggtgt cagaggtgaa attcttggat ttccgcaaga caaacagctg cgaaagcgtt     900
   tgccaaggat gctttcgtcg atcaagaacg aaagttgggg gctcgaagac gattagatac     960
   cgtcctagtc tcaaccataa acgatgccga ctagggatcg gcgagggttc tatcaacgac    1020
   ctcgccggca cctcatgaga aatcaaagtg tttgggttcc ggggggagta tggtcgcaag    1080
   gctgaaactt aaaggaattg acggaagggc accaccaggc gtggagcctg tggcttaatt    1140
   tgactcaaca cgggaaacct taccagtcca gacatagtga ggattgacag attgagagct    1200
   c                                                                    1201